# EUROPEAN PATENT APPLICATION

(11) **EP 1 739 168 A2**
(43) Date of publication of application: **03.01.2007**
(21) Application number: 06253346.8
(22) Date of filing: 27.06.2006
(51) Int. Cl.: C12N 9/02, C12N 15/53, C12Q 1/68

(54) **Neoplasia-specific splice variants and methods**

(30) Priority: 27.06.2005 US 167926
(71) Applicant: Purdue Research Foundation, West Lafayette, IN 47906 (US)
(72) Inventor: Morré, D. James, West Lafayette, IN 47906 (US)
(74) Representative: Lord, Hilton David

(57) **Abstract**

All neoplastic cells express a unique cell-surface ubiquinone (NADH) oxidase with protein disulfide-thiol isomerase with characteristic sensitivity to inhibition by capsaicin, sulfonylureas, adriamycin and certain other compounds. This neoplasia-specific protein is the translational expression production of a particular splice pattern: exon 4 is not translated to become part of the neoplasia-specific protein displayed on the surfaces of the cancer cells. Oligonucleotides which span the splice junctions of the cancer-specific mRNA can be used in RT-PCR assays, for example, having nucleotide sequences as given in SEQ ID NO:4 and in SEQ ID NO:5, which, when positive, are useful in the detection of neoplastic cells in the sample from which the mRNA was derived. Alternatively, transcriptase or real time polymerase chain reaction assays can produce amplification products of cancer-specific sizes. In addition, antisense oligonucleotides which inhibit the expression of the neoplasia-specific transcript are disclosed; these restore the normal phenotype of neoplastic cells into which they are introduced.

SEQUENCE LISTING

<110> Purdue Research Foundation

<120> Neoplasia-specific Splice Variants and Methods

<130> EPP95236

<150> US 11/167,926
<151> 2005-06-27

<160> 27

<170> PatentIn version 3.3

<210> 1
<211> 24
<212> DNA
<213> Artificial

<220>
<223> oligonucleotide useful as a primer

<400> 1
cagccgataa cagtagaact ctga 24

<210> 2
<211> 27
<212> DNA
<213> Artificial

<220>
<223> Oligonucleotide useful as a primer

<400> 2
ctgattcctc agtttctttt gtttctg 27

<210> 3
<211> 19

<212>  DNA
<213>  Artificial

<220>
<223>  oligonucleotide useful as a primer.

<400>  3
ggtaaaattg gtgtccggc                                                    19


<210>  4
<211>  20
<212>  DNA
<213>  Artificial

<220>
<223>  Oligonucleotide useful as a primer.

<400>  4
tccctccaaa tccaataccg                                                   20


<210>  5
<211>  27
<212>  DNA
<213>  Artificial

<220>
<223>  Oligonucleotide useful as a primer.

<400>  5
ctgattcctc agtttctttt ctttctg                                           27


<210>  6
<211>  23
<212>  DNA
<213>  Artificial

<220>
<223>  Oligonucleotide useful as a primer.

<400> 6
ccgataacag tagaactctg aac 23

<210> 7
<211> 27
<212> DNA
<213> Artificial

<220>
<223> Oligonucleotide useful as a primer.

<400> 7
ggctgattcc tcagtttctt ttgtttc 27

<210> 8
<211> 17
<212> PRT
<213> Artificial

<220>
<223> Oligopeptide corresponding to tNOX adenine nucleotide binding
site.

<400> 8

Thr Gly Val Gly Ala Ser Leu Glu Lys Arg Trp Lys Phe Cys Gly Phe
1               5                   10                  15

Glu

<210> 9
<211> 26
<212> PRT
<213> Artificial

<220>
<223>  Oligopeptide corresponding to tNOX quinone binding site

<400>  9

Glu Glu Met Thr Glu Cys Arg Arg Glu Glu Glu Met Glu Met Ser Asp
1               5                   10                  15

Asp Glu Ile Glu Glu Met Thr Glu Thr Lys
            20                  25

<210>  10
<211>  3580
<212>  DNA
<213>  Homo sapiens

<400>  10
gttcacagtt gaggaccaca caatgcaaag agattttaga tggctgtggg tctacgaaat      60

aggctatgca gccgataaca gtagaactct gaacgtggat tccactgcaa tgacactacc     120

tatgtctgat ccaactgcat gggccacagc aatgaataat cttggaatgg caccgctggg     180

aattgccgga caaccaattt tacctgactt tgatcctgct cttggaatga tgactggaat     240

tccaccaata actccaatga tgcctggttt gggaatagta cctccaccaa ttcctccaga     300

tatgccagta gtaaaagaga tcatacactg taaaagctgc acgctcttcc ctccaaatcc     360

aataccgcat tcgcctgggc tctagtactg acaagaagga cacaggcaga ctccacgttg     420

atttcgcaca ggctcgagat gacctgtatg agtgggagtg taaacagcgt atgctagcca     480

gagaggagcg ccatcgtaga agaatggaag aagaaagatt gcgtccacca tctccacccc     540

cagtggtcca ctattcagat catgaatgca gcattgttgc tgaaaaatta aaagatgatt     600

ccaaattctc agaagctgta cagaccttgc ttacctggat agagcgagga gaggtcaacc     660

gtcgtagcgc caataacttc tactccatga tccagtcggc caacagccat gtccgccgcc 720

tggtgaacga gaaagctgcc catgagaaag atatggaaga agcaaaggag aagttcaagc 780

aggccctttc tggaattctc attcaatttg agcagatagt ggctgtgtac cattccgcct 840

ccaagcagaa ggcatgggac cacttcacaa aagcccagcg gaagaacatc agcgtgtggt 900

gcaaacaagc tgaggaaatt cgcaacattc ataatgatga attaatggga atcaggcgag 960

aagaagaaat ggaaatgtct gatgatgaaa tagaagaaat gacagaaaca aaagaaactg 1020

aggaatcagc cttagtatca caggcagaag ctctgaagga agaaaatgac agcctccgtt 1080

ggcagctcga tgcctaccgg aatgaagtag aactgctcaa gcaagaacaa ggcaaagtcc 1140

acagagaaga tgaccctaac aaagaacagc agctgaaact cctgcaacaa gccctgcaag 1200

gaatgcaaca gcatctactc aaagtccaag aggaatacaa aaagaaagaa gctgaacttg 1260

aaaaactcaa agatgacaag ttacaggtgg aaaaaatgtt ggaaaatctt aaagaaaagg 1320

aaagctgtgc ttctaggctg tgtgcctcaa accaggatag cgaataccct cttgagaaga 1380

ccatgaacag cagtcctatc aaatctgaac gtgaagcact gctagtgggg attatctcca 1440

cattccttca tgttcaccca tttggagcaa gcattgaata catctgttcc tacttgcacc 1500

gtcttgataa taagatctgc accagcgatg tggagtgtct catgggtaga ctccagcata 1560

ccttcaagca ggaaatgact ggagttggag ccagcctgga aaagagatgg aaattctgtg 1620

gcttcgaggg cttgaagctg acctaaatct ctttgcctaa caacttggga tcctgaagat 1680

aaatatgtgt tggacaagca tagaaagtga tttatatttt taatggtttt caagtggaag 1740

ttcctttgaa tttgtcagtt cattcctgga aaatcttttg agttaaaata aggatcctag 1800

gacagcacct cgaactacag gccctaaaga gaaattgcct caaaccacaa gtgctgtaac 1860

ttcctcccct ttctgtcaat tggttgtctt taaatattgc aaaagtcctg atgctaaaca 1920

gtatttggag tgttttcagt gtctgtacta ctgttgtaca ccttggtatt tttttaaaca 1980

ctgttaactg aaatgttttg atgattttat gtgatttgtg tttctaaact tctctttaca 2040

ttaatgttgt tactggtgaa aggcatgaga gcagcactaa gtcctctgtg taactgccat 2100

tgtctttcca atccccagta gaccagtaaa taaataacac atcagtgtct tctagaaggt 2160

gcctgaccag gttcaccttt taaacgacaa agcatggttt gtggcttttt gcaaaattac 2220

tatgaaccaa aagttgacaa atgttccaaa gttattttct ctaacatatc acattaaaga 2280

tctgtttcag aattgtaaaa agtacatcta gatgtgttta cagaaagcaa gtatccagta 2340

tgactggcat gtgttcatgc tattcagaat cacttgtaaa tagtctgctt ttaaaggagg 2400

gcatgttcag ttttctgtga attaaaatat gctcatgtgt gggcacacac gcacaaacac 2460

acacacgcac gcacacagtg gcagaaggga tttatattaa tattctttcc cctctggcct 2520

tcttacagtc tgttggtccc tttgcttctg ttgtcagtgt gttgaattgc aaaccgagta 2580

ctgctgtaaa tactatgttt acttcatgct gaatgtttgc aaagacttga tataagtatt 2640

aatagtaatg aatcaatgaa taaataatga gctagggttt gtgaggcttt ctacaaatag 2700

gtcagctcca cctggagtgc gaattgccag agacaccttg gtagtgccca tcggcaaatc 2760

gcaatggcag catgtgagtg gaccattcag aaacttctgc ttggtggaaa gtaaacagag 2820

aggatggagg tttggggcga atgtcctgag gcagagatgg tctttattgt gtgtggtggt 2880

ggttgtggta tttataataa tgcaagcata ccctcccttg agtctcaatt gaagataaaa 2940

gaatgtactg agcaagcaaa gccaatggag agtatttcac aaaaatactt tgtaaatgag 3000

atgccagtag tgttcaaagt tgtattttta aaagataaat attccttttt atacctcagt 3060

tttgtgtcct gttttttaat gacttacgct ctaagtaatc cattagtagt tatctcagtc 3120

cctccctttg ggttactaga atgttggaaa aagatgccaa gtctgtcttg acaactggaa 3180

acagggttcc acagcagccc attcgtgctg aaaactggct tcccccctga agcaccctgc 3240

tgtggcacca gcaggaagct caggttaatt ttacactagc ttgctcactg atgcatctct 3300

catcaatgct acggaaggct ttgattcatc agtctcgggc tcttggaata cctaatttta 3360

ataatatcta tgaaatcaag ggaaactttc catttacagt tatttcttgt ttaaataaac 3420

taaattaatt tttaggggag agcagtagga aaaagagcta atgcatgcgg ggtttaatac 3480

ctaggtgatg ggttgaggtg cagcaaaacc accatggcac acgttcacct atgtaacaaa 3540

cctgcacatc ctgcacatgt accccggaac ttacttaaaa 3580

<210> 11
<211> 3555
<212> DNA
<213> Homo sapiens

<400> 11
gttcacagtt gaggaccaca caatgcaaag agattttaga tggctgtggg tctacgaaat 60

aggctatgca gccgataaca gtagaactct gaacgtggat tccactgcaa tgacactacc 120

tatgtctgat ccaactgcat gggccacagc aatgaataat cttggaatgg caccgctggg 180

aattgccgga caaccaattt tacctgactt tgatcctgct cttggaatga tgactggaat 240

tccaccaata actccaatga tgcctggttt gggaatagta cctccaccaa ttcctccaga 300

tatgccagta gtaaaagaga tcatacactg taaaagctgc acgctcttcc ctccaaatcc 360

aaatctccca cctcctgcaa cccgagaaag accaccagga tgcaaaacag tatttgtggg 420

tggtctgcct gaaaatggga cagagcaaat cattgtggaa gttttcgagc agtgtggaga 480

gatcattgcc attcgcaaga gcaagaagaa cttctgccac attcgctttg ctgaggagta 540

catggtggac aaagccctgt atctgtctgg tgattccaaa ttctcagaag ctgtacagac 600

cttgcttacc tggatagagc gaggagaggt caaccgtcgt agcgccaata acttctactc 660

catgatccag tcggccaaca gccatgtccg ccgcctggtg aacgagaaag ctgcccatga 720

gaaagatatg gaagaagcaa aggagaagtt caagcaggcc ctttctggaa ttctcattca 780

atttgagcag atagtggctg tgtaccattc cgcctccaag cagaaggcat gggaccactt 840

cacaaaagcc cagcggaaga acatcagcgt gtggtgcaaa caagctgagg aaattcgcaa 900

cattcataat gatgaattaa tgggaatcag gcgagaagaa gaaatggaaa tgtctgatga 960

tgaaatagaa gaaatgacag aaacaaaaga aactgaggaa tcagccttag tatcacaggc 1020

agaagctctg aaggaagaaa atgacagcct ccgttggcag ctcgatgcct accggaatga 1080

agtagaactg ctcaagcaag aacaaggcaa agtccacaga gaagatgacc ctaacaaaga 1140

acagcagctg aaactcctgc aacaagccct gcaaggaatg caacagcatc tactcaaagt 1200

ccaagaggaa tacaaaaaga aagaagctga acttgaaaaa ctcaaagatg acaagttaca 1260

ggtggaaaaa atgttggaaa atcttaaaga aaaggaaagc tgtgcttcta ggctgtgtgc 1320

ctcaaaccag gatagcgaat accctcttga gaagaccatg aacagcagtc ctatcaaatc 1380

tgaacgtgaa gcactgctag tggggattat ctccacattc cttcatgttc acccatttgg 1440

agcaagcatt gaatacatct gttcctactt gcaccgtctt gataataaga tctgcaccag 1500

cgatgtggag tgtctcatgg gtagactcca gcataccttc aagcaggaaa tgactggagt 1560

tggagccagc ctggaaaaga gatggaaatt ctgtggcttc gagggcttga agctgaccta 1620

aatctctttg cctaacaact tgggatcctg aagataaata tgtgttggac aagcatagaa 1680

agtgatttat atttttaatg gttttcaagt ggaagttcct ttgaatttgt cagttcattc 1740

ctggaaaatc ttttgagtta aaataaggat cctaggacag cacctcgaac tacaggccct 1800

aaagagaaat tgcctcaaac cacaagtgct gtaacttcct cccctttctg tcaattggtt 1860

gtctttaaat attgcaaaag tcctgatgct aaacagtatt tggagtgttt tcagtgtctg 1920

tactactgtt gtacaccttg gtattttttt aaacactgtt aactgaaatg ttttgatgat 1980

tttatgtgat ttgtgtttct aaacttctct ttacattaat gttgttactg gtgaaaggca 2040

tgagagcagc actaagtcct ctgtgtaact gccattgtct ttccaatccc cagtagacca 2100

gtaaataaat aacacatcag tgtcttctag aaggtgcctg accaggttca ccttttaaac 2160

gacaaagcat ggtttgtggc tttttgcaaa attactatga accaaaagtt gacaaatgtt 2220

ccaaagttat tttctctaac atatcacatt aaagatctgt ttcagaattg taaaaagtac 2280

atctagatgt gtttacagaa agcaagtatc cagtatgact ggcatgtgtt catgctattc 2340

agaatcactt gtaaatagtc tgcttttaaa ggagggcatg ttcagttttc tgtgaattaa 2400

aatatgctca tgtgtgggca cacacgcaca aacacacaca cgcacgcaca cagtggcaga 2460

agggatttat attaatattc tttcccctct ggccttctta cagtctgttg gtccctttgc 2520

ttctgttgtc agtgtgttga attgcaaacc gagtactgct gtaaatacta tgtttacttc 2580

atgctgaatg tttgcaaaga cttgatataa gtattaatag taatgaatca atgaataaat 2640

aatgagctag ggtttgtgag gctttctaca aataggtcag ctccacctgg agtgcgaatt 2700

gccagagaca ccttggtagt gcccatcggc aaatcgcaat ggcagcatgt gagtggacca 2760

ttcagaaact tctgcttggt ggaaagtaaa cagagaggat ggaggtttgg ggcgaatgtc 2820

ctgaggcaga gatggtcttt attgtgtgtg gtggtggttg tggtatttat aataatgcaa 2880

gcataccctc ccttgagtct caattgaaga taaaagaatg tactgagcaa gcaaagccaa 2940

tggagagtat ttcacaaaaa tactttgtaa atgagatgcc agtagtgttc aaagttgtat 3000

ttttaaaaga taaatattcc tttttatacc tcagttttgt gtcctgtttt ttaatgactt 3060

acgctctaag taatccatta gtagttatct cagtccctcc ctttgggtta ctagaatgtt 3120

ggaaaaagat gccaagtctg tcttgacaac tggaaacagg gttccacagc agcccattcg 3180

tgctgaaaac tggcttcccc cctgaagcac cctgctgtgg caccagcagg aagctcaggt 3240

taattttaca ctagcttgct cactgatgca tctctcatca atgctacgga aggctttgat 3300

tcatcagtct cgggctcttg gaatacctaa ttttaataat atctatgaaa tcaagggaaa 3360

ctttccattt acagttattt cttgtttaaa taaactaaat taatttttag gggagagcag 3420

taggaaaaag agctaatgca tgcggggttt aatacctagg tgatgggttg aggtgcagca 3480

aaaccaccat ggcacacgtt cacctatgta acaaacctgc acatcctgca catgtacccc 3540

ggaacttact taaaa 3555

<210> 12
<211> 3789
<212> DNA
<213> Homo sapiens

<220>
<221> CDS
<222> (23)..(1852)

<400> 12
gttcacagtt gaggaccaca ca atg caa aga gat ttt aga tgg ctg tgg gtc 52
Met Gln Arg Asp Phe Arg Trp Leu Trp Val
1               5                   10

tac gaa ata ggc tat gca gcc gat aac agt aga act ctg aac gtg gat 100
Tyr Glu Ile Gly Tyr Ala Ala Asp Asn Ser Arg Thr Leu Asn Val Asp
                15                  20                  25

tcc act gca atg aca cta cct atg tct gat cca act gca tgg gcc aca 148

Ser Thr Ala Met Thr Leu Pro Met Ser Asp Pro Thr Ala Trp Ala Thr
            30                  35                  40

gca atg aat aat ctt gga atg gca ccg ctg gga att gcc gga caa cca 196
Ala Met Asn Asn Leu Gly Met Ala Pro Leu Gly Ile Ala Gly Gln Pro
        45                  50                  55

att tta cct gac ttt gat cct gct ctt gga atg atg act gga att cca 244
Ile Leu Pro Asp Phe Asp Pro Ala Leu Gly Met Met Thr Gly Ile Pro
    60                  65                  70

cca ata act cca atg atg cct ggt ttg gga ata gta cct cca cca att 292
Pro Ile Thr Pro Met Met Pro Gly Leu Gly Ile Val Pro Pro Pro Ile
75                  80                  85                  90

cct cca gat atg cca gta gta aaa gag atc ata cac tgt aaa agc tgc 340
Pro Pro Asp Met Pro Val Val Lys Glu Ile Ile His Cys Lys Ser Cys
                95                  100                 105

acg ctc ttc cct cca aat cca aat ctc cca cct cct gca acc cga gaa 388
Thr Leu Phe Pro Pro Asn Pro Asn Leu Pro Pro Pro Ala Thr Arg Glu
            110                 115                 120

aga cca cca gga tgc aaa aca gta ttt gtg ggt ggt ctg cct gaa aat 436
Arg Pro Pro Gly Cys Lys Thr Val Phe Val Gly Gly Leu Pro Glu Asn
        125                 130                 135

ggg aca gag caa atc att gtg gaa gtt ttc gag cag tgt gga gag atc 484
Gly Thr Glu Gln Ile Ile Val Glu Val Phe Glu Gln Cys Gly Glu Ile
    140                 145                 150

att gcc att cgc aag agc aag aag aac ttc tgc cac att cgc ttt gct 532
Ile Ala Ile Arg Lys Ser Lys Lys Asn Phe Cys His Ile Arg Phe Ala
155                 160                 165                 170

gag gag tac atg gtg gac aaa gcc ctg tat ctg tct ggt tac cgc att 580
Glu Glu Tyr Met Val Asp Lys Ala Leu Tyr Leu Ser Gly Tyr Arg Ile
                175                 180                 185

cgc ctg ggc tct agt act gac aag aag gac aca ggc aga ctc cac gtt 628
Arg Leu Gly Ser Ser Thr Asp Lys Lys Asp Thr Gly Arg Leu His Val

            190                 195                 200

gat ttc gca cag gct cga gat gac ctg tat gag tgg gag tgt aaa cag 676
Asp Phe Ala Gln Ala Arg Asp Asp Leu Tyr Glu Trp Glu Cys Lys Gln
        205                 210                 215

cgt atg cta gcc aga gag gag cgc cat cgt aga aga atg gaa gaa gaa 724
Arg Met Leu Ala Arg Glu Glu Arg His Arg Arg Arg Met Glu Glu Glu
    220                 225                 230

aga ttg cgt cca cca tct cca ccc cca gtg gtc cac tat tca gat cat 772
Arg Leu Arg Pro Pro Ser Pro Pro Pro Val Val His Tyr Ser Asp His
235                 240                 245                 250

gaa tgc agc att gtt gct gaa aaa tta aaa gat gat tcc aaa ttc tca 820
Glu Cys Ser Ile Val Ala Glu Lys Leu Lys Asp Asp Ser Lys Phe Ser
                255                 260                 265

gaa gct gta cag acc ttg ctt acc tgg ata gag cga gga gag gtc aac 868
Glu Ala Val Gln Thr Leu Leu Thr Trp Ile Glu Arg Gly Glu Val Asn
            270                 275                 280

cgt cgt agc gcc aat aac ttc tac tcc atg atc cag tcg gcc aac agc 916
Arg Arg Ser Ala Asn Asn Phe Tyr Ser Met Ile Gln Ser Ala Asn Ser
        285                 290                 295

cat gtc cgc cgc ctg gtg aac gag aaa gct gcc cat gag aaa gat atg 964
His Val Arg Arg Leu Val Asn Glu Lys Ala Ala His Glu Lys Asp Met
    300                 305                 310

gaa gaa gca aag gag aag ttc aag cag gcc ctt tct gga att ctc att 1012
Glu Glu Ala Lys Glu Lys Phe Lys Gln Ala Leu Ser Gly Ile Leu Ile
315                 320                 325                 330

caa ttt gag cag ata gtg gct gtg tac cat tcc gcc tcc aag cag aag 1060
Gln Phe Glu Gln Ile Val Ala Val Tyr His Ser Ala Ser Lys Gln Lys
                335                 340                 345

gca tgg gac cac ttc aca aaa gcc cag cgg aag aac atc agc gtg tgg 1108
Ala Trp Asp His Phe Thr Lys Ala Gln Arg Lys Asn Ile Ser Val Trp
            350                 355                 360

tgc aaa caa gct gag gaa att cgc aac att cat aat gat gaa tta atg     1156
Cys Lys Gln Ala Glu Glu Ile Arg Asn Ile His Asn Asp Glu Leu Met
        365                 370                 375

gga atc agg cga gaa gaa gaa atg gaa atg tct gat gat gaa ata gaa     1204
Gly Ile Arg Arg Glu Glu Glu Met Glu Met Ser Asp Asp Glu Ile Glu
    380                 385                 390

gaa atg aca gaa aca aaa gaa act gag gaa tca gcc tta gta tca cag     1252
Glu Met Thr Glu Thr Lys Glu Thr Glu Glu Ser Ala Leu Val Ser Gln
395                 400                 405                 410

gca gaa gct ctg aag gaa gaa aat gac agc ctc cgt tgg cag ctc gat     1300
Ala Glu Ala Leu Lys Glu Glu Asn Asp Ser Leu Arg Trp Gln Leu Asp
                415                 420                 425

gcc tac cgg aat gaa gta gaa ctg ctc aag caa gaa caa ggc aaa gtc     1348
Ala Tyr Arg Asn Glu Val Glu Leu Leu Lys Gln Glu Gln Gly Lys Val
            430                 435                 440

cac aga gaa gat gac cct aac aaa gaa cag cag ctg aaa ctc ctg caa     1396
His Arg Glu Asp Asp Pro Asn Lys Glu Gln Gln Leu Lys Leu Leu Gln
        445                 450                 455

caa gcc ctg caa gga atg caa cag cat cta ctc aaa gtc caa gag gaa     1444
Gln Ala Leu Gln Gly Met Gln Gln His Leu Leu Lys Val Gln Glu Glu
    460                 465                 470

tac aaa aag aaa gaa gct gaa ctt gaa aaa ctc aaa gat gac aag tta     1492
Tyr Lys Lys Lys Glu Ala Glu Leu Glu Lys Leu Lys Asp Asp Lys Leu
475                 480                 485                 490

cag gtg gaa aaa atg ttg gaa aat ctt aaa gaa aag gaa agc tgt gct     1540
Gln Val Glu Lys Met Leu Glu Asn Leu Lys Glu Lys Glu Ser Cys Ala
                495                 500                 505

tct agg ctg tgt gcc tca aac cag gat agc gaa tac cct ctt gag aag     1588
Ser Arg Leu Cys Ala Ser Asn Gln Asp Ser Glu Tyr Pro Leu Glu Lys
            510                 515                 520

acc atg aac agc agt cct atc aaa tct gaa cgt gaa gca ctg cta gtg 1636
Thr Met Asn Ser Ser Pro Ile Lys Ser Glu Arg Glu Ala Leu Leu Val
        525                 530                 535

ggg att atc tcc aca ttc ctt cat gtt cac cca ttt gga gca agc att 1684
Gly Ile Ile Ser Thr Phe Leu His Val His Pro Phe Gly Ala Ser Ile
    540                 545                 550

gaa tac atc tgt tcc tac ttg cac cgt ctt gat aat aag atc tgc acc 1732
Glu Tyr Ile Cys Ser Tyr Leu His Arg Leu Asp Asn Lys Ile Cys Thr
555                 560                 565                 570

agc gat gtg gag tgt ctc atg ggt aga ctc cag cat acc ttc aag cag 1780
Ser Asp Val Glu Cys Leu Met Gly Arg Leu Gln His Thr Phe Lys Gln
                575                 580                 585

gaa atg act gga gtt gga gcc agc ctg gaa aag aga tgg aaa ttc tgt 1828
Glu Met Thr Gly Val Gly Ala Ser Leu Glu Lys Arg Trp Lys Phe Cys
            590                 595                 600

ggc ttc gag ggc ttg aag ctg acc taaatctctt tgcctaacaa cttgggatcc 1882
Gly Phe Glu Gly Leu Lys Leu Thr
        605                 610

tgaagataaa tatgtgttgg acaagcatag aaagtgattt atatttttaa tggttttcaa 1942

gtggaagttc ctttgaattt gtcagttcat tcctggaaaa tcttttgagt taaaataagg 2002

atcctaggac agcacctcga actacaggcc ctaaagagaa attgcctcaa accacaagtg 2062

ctgtaacttc ctcccctttc tgtcaattgg ttgtctttaa atattgcaaa agtcctgatg 2122

ctaaacagta tttggagtgt tttcagtgtc tgtactactg ttgtacacct tggtattttt 2182

ttaaacactg ttaactgaaa tgttttgatg attttatgtg atttgtgttt ctaaacttct 2242

ctttacatta atgttgttac tggtgaaagg catgagagca gcactaagtc ctctgtgtaa 2302

ctgccattgt ctttccaatc cccagtagac cagtaaataa ataacacatc agtgtcttct 2362

agaaggtgcc tgaccaggtt caccttttaa acgacaaagc atggtttgtg gctttttgca 2422

aaattactat gaaccaaaag ttgacaaatg ttccaaagtt attttctcta acatatcaca 2482

ttaaagatct gtttcagaat tgtaaaaagt acatctagat gtgtttacag aaagcaagta 2542

tccagtatga ctggcatgtg ttcatgctat tcagaatcac ttgtaaatag tctgctttta 2602

aaggagggca tgttcagttt tctgtgaatt aaaatatgct catgtgtggg cacacacgca 2662

caaacacaca cacgcacgca cacagtggca gaagggattt atattaatat tctttcccct 2722

ctggccttct tacagtctgt tggtcccttt gcttctgttg tcagtgtgtt gaattgcaaa 2782

ccgagtactg ctgtaaatac tatgtttact tcatgctgaa tgtttgcaaa gacttgatat 2842

aagtattaat agtaatgaat caatgaataa ataatgagct agggtttgtg aggctttcta 2902

caaataggtc agctccacct ggagtgcgaa ttgccagaga caccttggta gtgcccatcg 2962

gcaaatcgca atggcagcat gtgagtggac cattcagaaa cttctgcttg gtggaaagta 3022

aacagagagg atggaggttt ggggcgaatg tcctgaggca gagatggtct ttattgtgtg 3082

tggtggtggt tgtggtattt ataataatgc aagcataccc tcccttgagt ctcaattgaa 3142

gataaaagaa tgtactgagc aagcaaagcc aatggagagt atttcacaaa aatactttgt 3202

aaatgagatg ccagtagtgt tcaaagttgt atttttaaaa gataaatatt cctttttata 3262

cctcagtttt gtgtcctgtt ttttaatgac ttacgctcta agtaatccat tagtagttat 3322

ctcagtccct ccctttgggt tactagaatg ttggaaaaag atgccaagtc tgtcttgaca 3382

actggaaaca gggttccaca gcagcccatt cgtgctgaaa actggcttcc cccctgaagc 3442

accctgctgt ggcaccagca ggaagctcag gttaatttta cactagcttg ctcactgatg 3502

catctctcat caatgctacg gaaggctttg attcatcagt ctcgggctct tggaatacct 3562

aattttaata atatctatga aatcaaggga aactttccat ttacagttat ttcttgttta 3622

aataaactaa attaattttt aggggagagc agtaggaaaa agagctaatg catgcggggt 3682

ttaataccta ggtgatgggt tgaggtgcag caaaaccacc atggcacacg ttcacctatg 3742

taacaaacct gcacatcctg cacatgtacc ccggaactta cttaaaa 3789

<210> 13
<211> 610
<212> PRT
<213> Homo sapiens

<400> 13

Met Gln Arg Asp Phe Arg Trp Leu Trp Val Tyr Glu Ile Gly Tyr Ala
1               5                   10                  15

Ala Asp Asn Ser Arg Thr Leu Asn Val Asp Ser Thr Ala Met Thr Leu
            20                  25                  30

Pro Met Ser Asp Pro Thr Ala Trp Ala Thr Ala Met Asn Asn Leu Gly
        35                  40                  45

Met Ala Pro Leu Gly Ile Ala Gly Gln Pro Ile Leu Pro Asp Phe Asp
    50                  55                  60

Pro Ala Leu Gly Met Met Thr Gly Ile Pro Pro Ile Thr Pro Met Met
65                  70                  75                  80

Pro Gly Leu Gly Ile Val Pro Pro Pro Ile Pro Pro Asp Met Pro Val
                85                  90                  95

Val Lys Glu Ile Ile His Cys Lys Ser Cys Thr Leu Phe Pro Pro Asn
            100                 105                 110

Pro Asn Leu Pro Pro Pro Ala Thr Arg Glu Arg Pro Pro Gly Cys Lys
        115                 120                 125

Thr Val Phe Val Gly Gly Leu Pro Glu Asn Gly Thr Glu Gln Ile Ile
    130                 135                 140

Val Glu Val Phe Glu Gln Cys Gly Glu Ile Ile Ala Ile Arg Lys Ser
145                 150                 155                 160

Lys Lys Asn Phe Cys His Ile Arg Phe Ala Glu Glu Tyr Met Val Asp
                165                 170                 175

Lys Ala Leu Tyr Leu Ser Gly Tyr Arg Ile Arg Leu Gly Ser Ser Thr
            180                 185                 190

Asp Lys Lys Asp Thr Gly Arg Leu His Val Asp Phe Ala Gln Ala Arg
        195                 200                 205

Asp Asp Leu Tyr Glu Trp Glu Cys Lys Gln Arg Met Leu Ala Arg Glu
    210                 215                 220

Glu Arg His Arg Arg Arg Met Glu Glu Glu Arg Leu Arg Pro Pro Ser
225                 230                 235                 240

Pro Pro Pro Val Val His Tyr Ser Asp His Glu Cys Ser Ile Val Ala
                245                 250                 255

Glu Lys Leu Lys Asp Asp Ser Lys Phe Ser Glu Ala Val Gln Thr Leu
            260                 265                 270

Leu Thr Trp Ile Glu Arg Gly Glu Val Asn Arg Arg Ser Ala Asn Asn
        275                 280                 285

Phe Tyr Ser Met Ile Gln Ser Ala Asn Ser His Val Arg Arg Leu Val
    290                 295                 300

Asn Glu Lys Ala Ala His Glu Lys Asp Met Glu Glu Ala Lys Glu Lys
305                 310                 315                 320

Phe Lys Gln Ala Leu Ser Gly Ile Leu Ile Gln Phe Glu Gln Ile Val
                325                 330                 335

Ala Val Tyr His Ser Ala Ser Lys Gln Lys Ala Trp Asp His Phe Thr
            340                 345                 350

Lys Ala Gln Arg Lys Asn Ile Ser Val Trp Cys Lys Gln Ala Glu Glu
        355                 360                 365

Ile Arg Asn Ile His Asn Asp Glu Leu Met Gly Ile Arg Arg Glu Glu
    370                 375                 380

Glu Met Glu Met Ser Asp Asp Glu Ile Glu Glu Met Thr Glu Thr Lys
385                 390                 395                 400

Glu Thr Glu Glu Ser Ala Leu Val Ser Gln Ala Glu Ala Leu Lys Glu
                405                 410                 415

Glu Asn Asp Ser Leu Arg Trp Gln Leu Asp Ala Tyr Arg Asn Glu Val
            420                 425                 430

Glu Leu Leu Lys Gln Glu Gln Gly Lys Val His Arg Glu Asp Asp Pro

435                 440                 445

Asn Lys Glu Gln Gln Leu Lys Leu Leu Gln Gln Ala Leu Gln Gly Met
    450                 455                 460

Gln Gln His Leu Leu Lys Val Gln Glu Glu Tyr Lys Lys Lys Glu Ala
465                 470                 475                 480

Glu Leu Glu Lys Leu Lys Asp Asp Lys Leu Gln Val Glu Lys Met Leu
                485                 490                 495

Glu Asn Leu Lys Glu Lys Glu Ser Cys Ala Ser Arg Leu Cys Ala Ser
            500                 505                 510

Asn Gln Asp Ser Glu Tyr Pro Leu Glu Lys Thr Met Asn Ser Ser Pro
        515                 520                 525

Ile Lys Ser Glu Arg Glu Ala Leu Leu Val Gly Ile Ile Ser Thr Phe
    530                 535                 540

Leu His Val His Pro Phe Gly Ala Ser Ile Glu Tyr Ile Cys Ser Tyr
545                 550                 555                 560

Leu His Arg Leu Asp Asn Lys Ile Cys Thr Ser Asp Val Glu Cys Leu
                565                 570                 575

Met Gly Arg Leu Gln His Thr Phe Lys Gln Glu Met Thr Gly Val Gly
            580                 585                 590

Ala Ser Leu Glu Lys Arg Trp Lys Phe Cys Gly Phe Glu Gly Leu Lys
        595                 600                 605

Leu Thr
    610

<210>  14
<211>  391
<212>  PRT
<213>  Homo sapiens

<400>  14

Met Leu Ala Arg Glu Glu Arg His Arg Arg Arg Met Glu Glu Glu Arg
1               5                   10                  15

Leu Arg Pro Pro Ser Pro Pro Pro Val Val His Tyr Ser Asp His Glu
            20                  25                  30

Cys Ser Ile Val Ala Glu Lys Leu Lys Asp Asp Ser Lys Phe Ser Glu
        35                  40                  45

Ala Val Gln Thr Leu Leu Thr Trp Ile Glu Arg Gly Glu Val Asn Arg
    50                  55                  60

Arg Ser Ala Asn Asn Phe Tyr Ser Met Ile Gln Ser Ala Asn Ser His
65                  70                  75                  80

Val Arg Arg Leu Val Asn Glu Lys Ala Ala His Glu Lys Asp Met Glu
                85                  90                  95

Glu Ala Lys Glu Lys Phe Lys Gln Ala Leu Ser Gly Ile Leu Ile Gln
            100                 105                 110

Phe Glu Gln Ile Val Ala Val Tyr His Ser Ala Ser Lys Gln Lys Ala
        115                 120                 125

Trp Asp His Phe Thr Lys Ala Gln Arg Lys Asn Ile Ser Val Trp Cys
    130                 135                 140

Lys Gln Ala Glu Glu Ile Arg Asn Ile His Asn Asp Glu Leu Met Gly
145                 150                 155                 160

Ile Arg Arg Glu Glu Glu Met Glu Met Ser Asp Asp Glu Ile Glu Glu
                165                 170                 175

Met Thr Glu Thr Lys Glu Thr Glu Glu Ser Ala Leu Val Ser Gln Ala
            180                 185                 190

Glu Ala Leu Lys Glu Glu Asn Asp Ser Leu Arg Trp Gln Leu Asp Ala
        195                 200                 205

Tyr Arg Asn Glu Val Glu Leu Leu Lys Gln Glu Gln Gly Lys Val His
    210                 215                 220

Arg Glu Asp Asp Pro Asn Lys Glu Gln Gln Leu Lys Leu Leu Gln Gln
225                 230                 235                 240

Ala Leu Gln Gly Met Gln Gln His Leu Leu Lys Val Gln Glu Glu Tyr
                245                 250                 255

Lys Lys Lys Glu Ala Glu Leu Glu Lys Leu Lys Asp Asp Lys Leu Gln
            260                 265                 270

Val Glu Lys Met Leu Glu Asn Leu Lys Glu Lys Glu Ser Cys Ala Ser

        275                 280                 285

Arg Leu Cys Ala Ser Asn Gln Asp Ser Glu Tyr Pro Leu Glu Lys Thr
    290                 295                 300

Met Asn Ser Ser Pro Ile Lys Ser Glu Arg Glu Ala Leu Leu Val Gly
305                 310                 315                 320

Ile Ile Ser Thr Phe Leu His Val His Pro Phe Gly Ala Ser Ile Glu
                325                 330                 335

Tyr Ile Cys Ser Tyr Leu His Arg Leu Asp Asn Lys Ile Cys Thr Ser
            340                 345                 350

Asp Val Glu Cys Leu Met Gly Arg Leu Gln His Thr Phe Lys Gln Glu
        355                 360                 365

Met Thr Gly Val Gly Ala Ser Leu Glu Lys Arg Trp Lys Phe Cys Gly
    370                 375                 380

Phe Glu Gly Leu Lys Leu Thr
385                 390

<210> 15
<211> 380
<212> PRT
<213> Homo sapiens

<400> 15

Met Glu Glu Glu Arg Leu Arg Pro Pro Ser Pro Pro Pro Val Val His
1               5                   10                  15

Tyr Ser Asp His Glu Cys Ser Ile Val Ala Glu Lys Leu Lys Asp Asp
            20                  25                  30

Ser Lys Phe Ser Glu Ala Val Gln Thr Leu Leu Thr Trp Ile Glu Arg
        35                  40                  45

Gly Glu Val Asn Arg Arg Ser Ala Asn Asn Phe Tyr Ser Met Ile Gln
    50                  55                  60

Ser Ala Asn Ser His Val Arg Arg Leu Val Asn Glu Lys Ala Ala His
65                  70                  75                  80

Glu Lys Asp Met Glu Glu Ala Lys Glu Lys Phe Lys Gln Ala Leu Ser
                85                  90                  95

Gly Ile Leu Ile Gln Phe Glu Gln Ile Val Ala Val Tyr His Ser Ala
            100                 105                 110

Ser Lys Gln Lys Ala Trp Asp His Phe Thr Lys Ala Gln Arg Lys Asn
        115                 120                 125

Ile Ser Val Trp Cys Lys Gln Ala Glu Glu Ile Arg Asn Ile His Asn
    130                 135                 140

Asp Glu Leu Met Gly Ile Arg Arg Glu Glu Glu Met Glu Met Ser Asp
145                 150                 155                 160

Asp Glu Ile Glu Glu Met Thr Glu Thr Lys Glu Thr Glu Glu Ser Ala
                165                 170                 175

Leu Val Ser Gln Ala Glu Ala Leu Lys Glu Glu Asn Asp Ser Leu Arg
            180                 185                 190

Trp Gln Leu Asp Ala Tyr Arg Asn Glu Val Glu Leu Leu Lys Gln Glu
        195                 200                 205

Gln Gly Lys Val His Arg Glu Asp Asp Pro Asn Lys Glu Gln Gln Leu
    210                 215                 220

Lys Leu Leu Gln Gln Ala Leu Gln Gly Met Gln Gln His Leu Leu Lys
225                 230                 235                 240

Val Gln Glu Glu Tyr Lys Lys Lys Glu Ala Glu Leu Glu Lys Leu Lys
                245                 250                 255

Asp Asp Lys Leu Gln Val Glu Lys Met Leu Glu Asn Leu Lys Glu Lys
            260                 265                 270

Glu Ser Cys Ala Ser Arg Leu Cys Ala Ser Asn Gln Asp Ser Glu Tyr
        275                 280                 285

Pro Leu Glu Lys Thr Met Asn Ser Ser Pro Ile Lys Ser Glu Arg Glu
    290                 295                 300

Ala Leu Leu Val Gly Ile Ile Ser Thr Phe Leu His Val His Pro Phe
305                 310                 315                 320

Gly Ala Ser Ile Glu Tyr Ile Cys Ser Tyr Leu His Arg Leu Asp Asn
                325                 330                 335

Lys Ile Cys Thr Ser Asp Val Glu Cys Leu Met Gly Arg Leu Gln His

340                 345                 350

Thr Phe Lys Gln Glu Met Thr Gly Val Gly Ala Ser Leu Glu Lys Arg
        355                 360                 365

Trp Lys Phe Cys Gly Phe Glu Gly Leu Lys Leu Thr
    370                 375                 380

<210>  16
<211>  33
<212>  DNA
<213>  Artificial

<220>
<223>  Oligonucleotide useful as primer.

<400>  16
gatctcgagc tcaagcttga ccacacaatg caa                                33

<210>  17
<211>  33
<212>  DNA
<213>  Artificial

<220>
<223>  Oligonucleotide useful as a primer.

<400>  17
atcccgggcc cgcggtaccg tcagcttcaa gcc                                33

<210>  18
<211>  21
<212>  DNA
<213>  Artificial

<220>

<223>  Oligonucleotide used as antisense nucleic acid molecule.

<400>  18
gtccaccatg tactcctcag c                                                21

<210>  19
<211>  21
<212>  DNA
<213>  Artificial

<220>
<223>  Oligonucleotide useful as an antisense nucleic acid molecule.

<400>  19
agctctttcc gcccatcgaa c                                                21

<210>  20
<211>  21
<212>  DNA
<213>  Artificial

<220>
<223>  Oligonucleotide useful as an antisense nucleic acid molecule.

<400>  20
gtctgcctgt gtccttcttg t                                                21

<210>  21
<211>  21
<212>  DNA
<213>  Artificial

<220>
<223>  Oligonucleotide useful as an antisense molecule.

<400>  21
tggcgtttcc gttctcgtct t                                                21

<210>  22
<211>  24
<212>  DNA
<213>  Artificial

<220>
<223>  Oligonucleotide useful as a primer.

<400>  22
tcgctttgct gaggagtaca tggt                                              24


<210>  23
<211>  24
<212>  DNA
<213>  Artificial

<220>
<223>  Oligonucleotide useful as a primer.

<400>  23
tctgcctgtg tccttcttgt cagt                                              24


<210>  24
<211>  22
<212>  DNA
<213>  Artificial

<220>
<223>  Oligonucleotide useful as a primer.

<400>  24
ccaaatccaa taccgcattc gc                                                22


<210>  25
<211>  24
<212>  DNA
<213>  Artificial

<220>
<223>  Oligonucleotide useful as a primer.

<400>  25
agcatacgct gtttacactc ccac                                              24


<210>  26
<211>  21
<212>  DNA
<213>  Artificial

<220>
<223>  Oligonucleotide useful as a primer.

<400>  26
ctgtctggtg attccaaatt c                                                 21


<210>  27
<211>  24
<212>  DNA
<213>  Artificial

<220>
<223>  Oligonucleotide useful as a primer.

<400>  27
ttctcatggg cagctttctc gttc                                              24

## Description

The field of this invention is the area of molecular biology, in particular, as related to the molecular biology of neoplastic and diseased cells, as specifically related to splice variant transcripts for a cell surface marker characteristic of neoplastic and certain other diseased cell states.

There is a unique, growth-related family of cell surface hydroquinone or NADH oxidases with protein disulfide-thiol interchange activity referred to as ECTO-NOX proteins (for cell surface NADH oxidases) (1, 2). One member of the ECTO-NOX family, designated tNOX (for tumor associated) is specific to the surfaces of cancer cells and the sera of cancer patients (3, 4). The presence of the tNOX protein has been demonstrated for several human tumor tissues (mammary carcinoma, prostate cancer, neuroblastoma, colon carcinoma and melanoma) (5), and serum analyses suggest a much broader association with human cancer (6, 7).

NOX proteins are ectoproteins anchored in the outer leaflet of the plasma membrane (8). As is characteristic of other examples of ectoproteins (sialyl and galactosyl transferase, dipeptidylamino peptidase IV, etc.), the NOX proteins are shed. They appear in soluble form in conditioned media of cultured cells (5) and in patient sera (6, 7). The serum form of tNOX from cancer patients exhibits the same degree of drug responsiveness as does the membrane-associated form. Drug-responsive tNOX activities are seen in sera of a variety of human cancer patients, including patients with leukemia, lymphomas or solid tumors (prostate, breast, colon, lung, pancreas, ovarian, liver) (6, 7). An extreme stability and protease resistance of the tNOX protein (9) may help explain its ability to accumulate in sera of cancer patients to readily detectable levels. In contrast, no drug-responsive NOX activities have been found in the sera of healthy volunteers (6, 7).

While the basis for the cancer specificity of cell surface tNOX has not been determined, the concept is strongly supported by several lines of evidence. A drug responsive tNOX activity has been rigorously determined to be absent from plasma membranes of non-transformed human and animal cells and tissues (3). The tNOX proteins lack a transmembrane binding domain (10) and are released from the cell surface by brief treatment at low pH (9). A drug-responsive tNOX activity has not been detected in sera from healthy volunteers or patients with diseases other than cancer (6, 7). Several tNOX antisera have identified the immunoreactive band at 34 kDa (the processed molecular weight of the cell surface form of tNOX) with Western blot analysis or immunoprecipitation when using non-transformed cells and tissues or sera from healthy volunteers or patients with disorders other than cancers as antigen source (5, 10, 11). Those antisera include a monoclonal antibody (5), single-chain variable region fragment (scFv) recombinant antibody derived from DNA of the monoclonal hybridoma (5), polyclonal antisera to expressed tNOX (11) and polyclonal peptide antisera to the conserved adenine nucleotide binding region of tNOX (11).

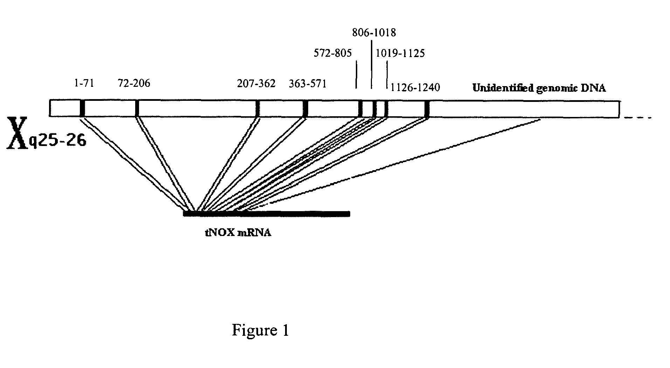

tNOX cDNA has been cloned (GenBank Accession No. AF207881; 11; U.S. Patent Publication 2003-0207340 A1). The derived molecular weight from the open reading frame was 70.1 kDa. Identified functional motifs include a quinone binding site, an adenine nucleotide binding site, and a CXXXXC cysteine pair as a potential protein disulfide-thiol interchange site based on site-directed mutagenesis (11). Based on available genomic information (12) the tNOX gene is located on chromosome X, and it is comprised of multiple exons (thirteen) (See Fig. 1).

Because cancer and certain viral, protozoan and parasite infections pose significant threats to human health and because cancer and such infections result in significant economic costs, there is a long-felt need in the art for an effective, economical and technically simple system in which to assay for or model for, identifying inhibitors of the aforementioned disease states.

The present invention provides a cancer-specific alternatively spliced tumor-specific plasma membrane NADH oxidase/thiol interchange protein transcript (termed E4mtNOX herein). This cDNA sequence, spliced such that exon 4 is not present, is shown in SEQ ID NO:10, and the resulting 44.2 kDa cancer-specific E4mtNOX-encoded primary translation product expressed therefrom (initiation at M231 of SEQ ID NO:13, the full length tNOX amino acid sequence, encoded by the DNA sequence of SEQ ID NO. 12) has the amino acid sequence given in SEQ ID NO:15. A second cancer-specific, alternatively spliced tNOX sequence, lacking exon 5 (E5mtNOX) has the coding sequence given in SEQ ID NO:11. Also within the scope of the present invention are coding sequences which are synonymous with the specifically exemplified E4mtNOX coding sequence. Also contemplated are sequences which encode a neoplastic cell surface marker and which coding sequences hybridize under stringent conditions to the specifically exemplified full length or partial sequence. The cell surface E4mtNOX protein is characteristic of neoplastic conditions, as are the particular mRNAs. The recombinant E4mtNOX protein is useful in preparing antigen for use in generation of monoclonal antibodies or antisera for diagnosis of neoplastic conditions, including cancers. The E4mtNOX protein is expressed in all cancerous (neoplastic cells), and thus, it can serve as a "pancancer" marker.

Within the scope of the present invention are recombinant DNA molecules comprising the alternately spliced coding sequence termed E4mtNOX herein, and optionally further comprising a heterologous sequence such as a tag sequence which facilitates purification. Such molecules may be non-naturally occurring or isolated. The sequence is preferably that of SEQ ID NO:10 or a homologue thereof or a complementary sequence thereto. The tag sequence can be, but is not limited to, a polyhistidine (His tag) sequence, a flagellar antigen tag sequence (Flag tag), a glutathione S-transferase tag sequence (GST tag) or biotin binding sequence (Strep tag). Also provided herein are methods for recombinantly producing a recombinant NADH oxidase/protein disulfide-thiol interchange active polypeptide in a host cell (bacterial, yeast, mammalian) using the alternately spliced sequence and recombinant DNA molecules provided herein.

The present invention further provides a method for determining neoplasia in a mammal, including a human, said method comprising the steps of detecting the presence, in a biological sample from a mammal, of an alternatively spliced NADH oxidase/protein disulfide thiol interchange protein ribonucleic acid molecule (lacking exon 4 or exon 5) uniquely associated with neoplastic cells as compared to a ribonucleic acid molecule encoding a NADH oxidase associated with normal cells. The step of detecting is carried out using hybridization under stringent conditions or using a polymerase chain reaction in which a perfect match of primer to template is required.A hybridization probe or forward RT-PCR primer comprising of at least 15 consecutive nucleotides as given in SEQ ID NO:4 or SEQ ID NO:6 is used to detect the cancer-specific splice variant. The presence of the ribonucleic acid molecule (lacking exon 4 or exon 5) in the biological sample is indicative of the presence of neoplasia. Homologues of SEQ ID NOS. 4 and 6 can also be used, as can sequences that are complementary thereto and bind thereto under stringent conditions, preferably highly stringent conditions, as discussed below. A preferred method is RT-PCR. These SEQ ID NO:4 and SEQ ID NO:6 probes and primers comprise sequences that encompass the splice junctions of the alternately spliced tNOX cDNA, as taught herein. Either of these forward primers can be used in conjunction with a primer downstream of the cancer-specific splice junction, for example a primer from exon 8, as specifically exemplified a primer characterized by the nucleotide sequence given in SEQ ID NO:5. Alternatively, a forward primer matching a sequence upstream of exon 4 or exon 5 can be used, with the size(s) of amplification products revealing whether the exon 4 or exon 5 sequences are missing from the transcript template.

The present invention further provides additional RT-PCT (reverse transcriptase polymerase chain reaction) methods for assessment of neoplasia in a patient sample, for example, in biopsy material, based on the cancer-specific transcription products corresponding in sequence to E4mtNOX and E5mtNOX. A forward primer is chosen to comprise a nucleotide sequence of at least 15, preferably 20-25 contiguous nucleotides, from the full length tNOX cDNA sequence SEQ ID NO:12, nucleotides 1-362, preferably from 1-330, when an E4tNOX or E5mtNOPX transcript is to be detected. The reverse primer is designed to contain at least 15, preferably 20-25, contiguous, complementary nucleotides (from SEQ ID NO:11) downstream of the missing exon 4 or exon 5 portions of the transcript to be detected, such that the amplification product is 208 nucleotides shorter in a cancer (neoplastic) cell or tissue than in a normal cell or tissue when the exon 4-minus product is detected and such that the amplification product is 233 nucleotides shorter when the exon 5-minus product is detected. A specifically exemplified reverse primer is given in SEQ ID NO:5; it is from exon 8. The use of a reverse primer downstream of both the exon 4 and exon 5 sequences allows the detection of both the E4mtNOX and E5tNOX transcripts in a single RT-PCR assay. Exon 4 corresponds to nucleotides 363 to 571 of SEQ ID NO:12, and exon 5 corresponds to nucleotides 572 to 805 of SEQ ID NO:12. Homologues of these primers and sequences complementary thereto are also preferred, as discussed below.

Also within the scope of the present invention are antisense oligonucleotides which block the expression of the cancer-specific tNOX protein and restore the normal phenotype of neoplastic cells into which one or more of those antisense oligonucleotides has been introduced. Specifically exemplified antisense nucleic acid molecules comprise sequences as set forth in SEQ ID NO:18, SEQ ID NO:19, SEQ ID NO:25 and SEQ ID NO:27. Again, homologues of these primers and sequences complementary thereto are also preferred.

Thus, it is noted that SEQ ID NO:4 is a DNA RT primer for Exon 4, and SEQ ID NO:6 is a DNA RT primer for Exon 5. Thus, these sequences are useful for detecting the presence or absence of these exons. They can also be used, for instance, in combination with SEQ ID NO:5, a DNA RT primer for Exon 8. Other primer sequences are also provided. SEQ ID NO:10 is the cDNA sequence for the E4-minus splice variant protein, i.e. the protein lacking Exon 4. SEQ ID NO:11 is the E5-minus splice variant cDNA sequence, lacking Exon 5. SEQ ID NO: 12 is the full length tNOX cDNA sequence, encoding the full length protein of SEQ ID NO:13. The E4-minus variant is translated as two translation products, SEQ ID NO:14 and SEQ ID NO:15, of 44 and 46 kDa respectively.

Where reference is made to homologues, it is preferred that the sequence has at least 80% sequence homology to the reference sequence, more preferably at least 85% sequence homology, more preferably at least 90% sequence homology, more preferably at least 95% sequence homology, more preferably at least 99% sequence homology, and most preferably at least 99.5% sequence homology to said reference sequence. As discussed below, this can be measured using standard methods in the art, such as the BLAST program. It will be appreciated by the skilled person that, where short sequences are referenced, that very high degrees of sequence homology may not be appropriate, due to the number of nucleotides available.

Also preferred are sequences complementary to the reference sequence or sequences having a specified degree of sequence homology thereto that bind under stringent conditions. Preferably, the conditions are moderately stringent, such as hybridizing at 68° C in 5X SSC/5X Denhardt's solution/0.1% SDS and washing at 42° C in 3X SSC, as discussed below. Alternative conditions of moderate stringency may include washing in 0.2 × SSC /0.1% SDS at 42 degrees C. (Ausubel F. M. et al., eds., 1989, Current Protocols in Molecular Biology, Vol. I, Green Publishing Associates, Inc., and John Wiley & Sons, Inc., NY, at p. 2.10.3)

More preferably, however, highly stringent conditions are used, such as hybridizing at 68° C in 5X SSC/5X Denhardt's solution/0.1% SDS, and washing in 0.2X SSC/0.1% SDS at room temperature. Alternatively, the highly stringent conditions may be hybridization to filter-bound DNA in 0.5 M NaHPO.sub.4, 7% sodium dodecyl sulfate (SDS), 1 mM EDTA at 65.degree. C., and washing in 0.1 × SSC /0.1% SDS at 68 degree. C. (Ausubel F. M. et al. *supra*),

Substitutions, insertions and deletions are also envisaged in the reference sequence, using, for instance, Dayhoff's rules, provided that the function is substantially retained.

Preferably, the polynucleotide is capable of hybridizing to the splice junctions of the alternatively spliced tNOX cDNA present within SEQ ID NO: 4 or SEQ ID NO: 6. The sequences are useful for detecting the presence of absence of the E4-minus or E5-minus splice variants indicative of the presence of absence, respectively, of neoplastic cells in a sample or a neoplastic condition. Thus, the sequences are for use in the methods described herein.

The invention also provides for probes comprising the primers of the present invention, preferably selected from the group consisting of SEQ ID NOS: 1-9 and 16-27. Preferably, the primers are SEQ ID NOS: 18, 20, 25, or 27, and most preferably SEQ ID NOS: 4 or 6. Preferably, the probes comprise a detectable marker or tag, as discussed above.

Also provided is a kit for detection of neoplastic cells, comprising the polynucleotides of the present invention. This is preferably a microarray, on which the polynucleotides, preferably the primers or probes of the present invention, are bound. Suitable methods of detection of binding will be apparent to the skilled person.

The present invention will now be described with reference to the accompanying figures, wherein:

Figure 1 is a schematic representation of the tNOX gene, which is located on the X chromosome. It is composed of 13 exons. The nucleotide numbers refer to SEQ ID NO:12.

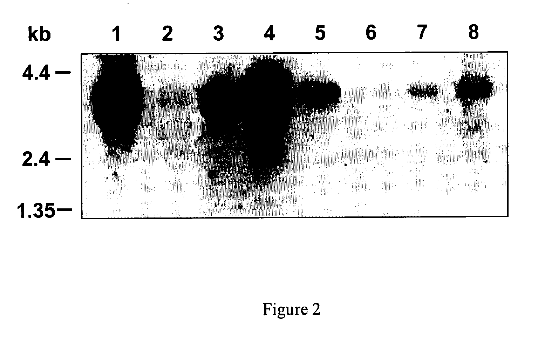

Figure 2 shows the results of Northern blot analysis of normal human tissues probed with a nucleic acid molecule corresponding in sequence to nucleotides 660-1878 of SEQ ID NO:12, the full length tNOX transcript. Similar results were obtained from probing with exon 4 (nucleotides 363-571 of SEQ ID NO:12) of tNOX. Lane 1: spleen. Lane 2: thymus. Lane 3: prostate. Lane 4: testis. Lane 5: ovary. Lane 6: small intestine. Lane 7: colon. Lane 8: peripheral blood leukocyte.

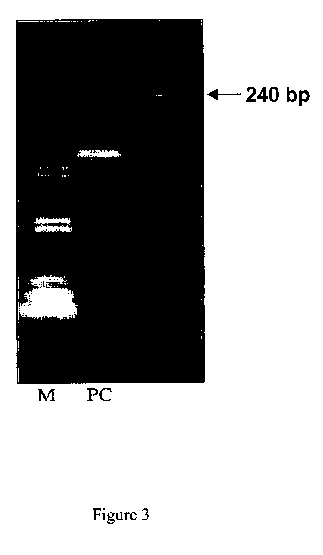

Figure 3 shows the verification of 5' terminus by 5' RACE using an exon 2 3' primer. The 240 bp band shown here corresponds to exon 1 plus exon 2 of tNOX as initially reported (6).

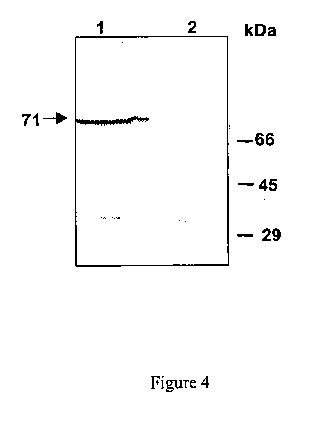

Figure 4 shows that the full-length tNOX cDNA expressed a protein around 71 kDa under the control of vector promoter in COS cells. Lane 1: full length tNOX cDNA in pcDNA 3.1. Lane 2: pcDNA 3.1. All are COS cell transfectants. Antibody PU04, a peptide antibody to the quinone binding region on tNOX was utilized.

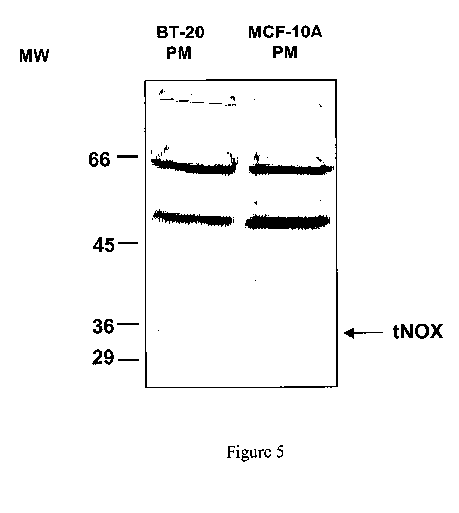

Figures 5A and 5B show the results of BT-20 (human mammary adenocarcinoma) and MCF-10A (non-cancer mammary epithelia) plasma membranes (0.05 mg protein per lane) analyzed by SDS-PAGE (10% gels) (Fig. 5A) and by Western blot analysis (Fig. 5B) with peptide antibody specific to conserved adenine nucleotide binding region of tNOX (PU04) showed the 34 kDa protein (arrow) present in the plasma membranes of the BT-20 cells was absent from plasma membranes of MCF-10A cells.

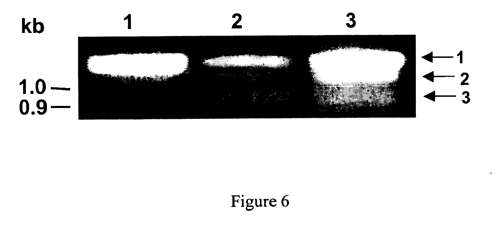

Figure 6 shows the results of RT-PCR of MCF-10A, BT-20 and HeLa cells. This image shows that in addition to tNOX mRNA (band 1), an exon 4 minus form (band 2) and an exon 5 minus product (band 3) in both cancer lines BT-20 (lane 2) and HeLa (lane 3) but not in the non-cancer MCF-10A (lane 1) cells.

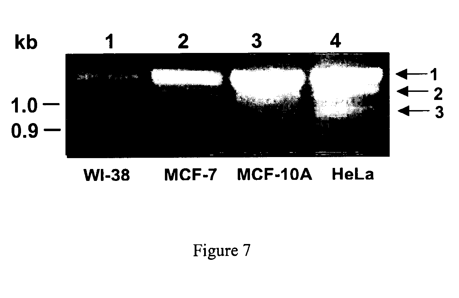

Figure 7 shows the results of RT-PCR for MCF-10A, MCF-7, WI-38 and HeLa cells. This image shows an exon 4 minus form (band 2) and an exon 5 minus form product (band 3) in both MCF-7 (lane 2) and HeLa (lane 4) cancer cells but not in non-cancer WI-38 (lane 1) and MCF-10A (lane 3) cells. The tNOX mRNA (band 1) was present in all 4 cell lines.

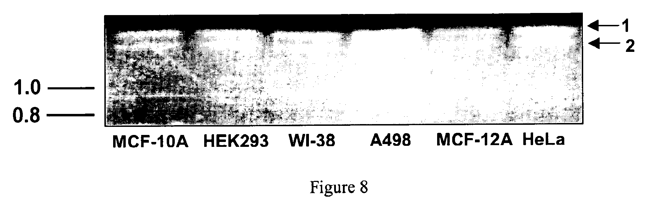

Figure 8 illustrates the results of RT-PCR, showing additional examples of alternative splicing with variations in exon 1. Primers used were nucleotides 191-215 of AK000353 (band 1) and DBS 3' (Drug binding site of tNOX 3' end) (band 2). In contrast to the Exon 4 minus form, these splice variants appear in both cancer (HEK 293, A498, MCF-12A, HeLa) and non-cancer (MCF-10A, WI-38) cell lines.

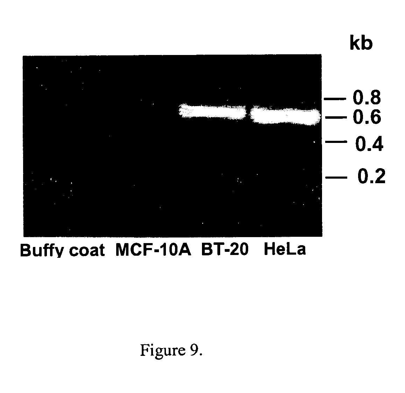

Figure 9 shows the results of RT-PCR analysis of exon 4 minus mRNA using a co-mer forward primer at the junction between exon 3 and exon 5 (primer sequence, SEQ ID NO:4) with the reverse primer being the end of exon 8 (primer sequence, SEQ ID NO:5). A single cancer-specific dominant species was generated (BT-20 and HeLa cells).

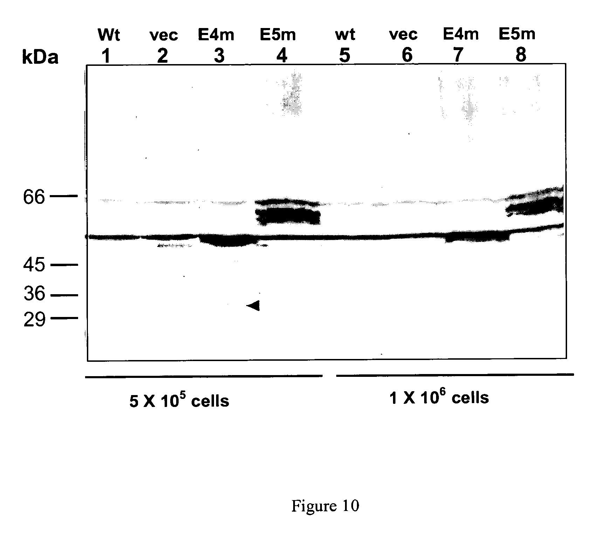

Figure 10 demonstrates expression of the Exon 4 minus form and the Exon 5 minus form in COS cells. lmmunostaining of Exon 4 minus form and Exon 5 minus form of tNOX in COS cells identified by tNOX peptide antibody to the C-terminal adenine binding motif. Lane 1 and 5, wild type COS. Lane 2 and 6, pcDNA3.1 vector. Lane 3 and 7, Exon 4 minus form tNOX. Lane 4 and 8, Exon 5 minus form tNOX.

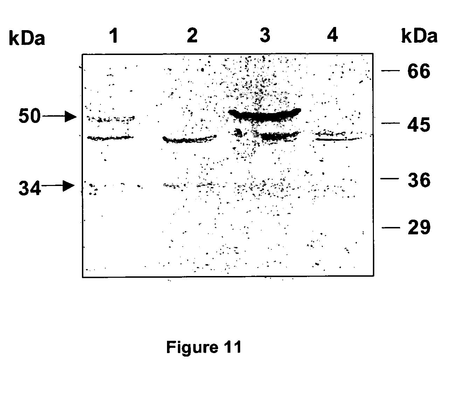

Figure 11 shows the results of a Western blot analysis of plasma membranes and internal membranes of the exon 4 minus transfectants and whole cell preparations probed by a peptide antibody toward the quinone binding motif of the tNOX protein. Lane 1: internal membranes of exon 4 minus variant transfectants. Lane 2: plasma membrane of exon 4 minus variant transfectants. Lane 3: whole cell protein of exon 4 minus variant transfectants. Lane 4: vector only transfectants.

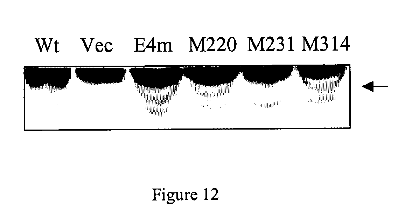

Figure 12 demonstrates that mutation of Met 231 prevents the protein expression of exon 4 minus mRNA. Lane 1: wild type COS cells Lane. 2: COS cells transfected with pcDNA 3.1 vector. Lane 3: COS cells transfected with exon 4 minus cDNA in pcDNA 3.1. Lane 4: COS cells transfected with mutant M220A exon 4 minus cDNA in pcDNA 3.1. Lane 5: COS cells transfected with mutant M231A exon 4 minus cDNA in pcDNA 3.1. Lane 6: COS cells transfected with mutant M314A exon 4 minus cDNA in pcDNA 3.1.

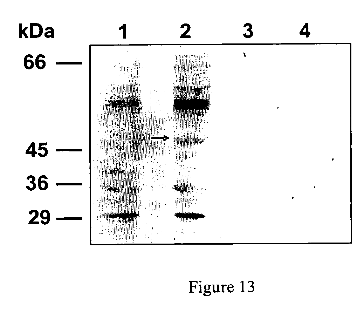

Figure 13 shows the results of immunoprecipitation of HeLa S cells with ScFv-Fc and detected with anti-fc antibody. Lane 1: plasma membrane of HeLa S cells immunoprecipitated with ScFv-Fc and detected with anti-fc antibody. Lane 2: endoplasmic reticulum of HeLa S cells immuno-precipitated with ScFv-Fc and detected with anti-fc antibody. Lane 3: plasma membrane of HeLa S cells immunoprecipitated with and detected with anti-fc antibody. Lane 4: endoreticulum of HeLa S cells immunoprecipitated with detected with anti-fc antibody. Arrow denotes an endoplasmic reticulum-specific 47 kDa band corresponding to the full length translation product predicted for initiation at Met 231.

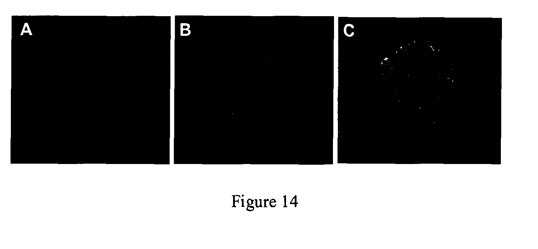

Figure 14 provides confocal microscope images of full length tNOX-EGFP transfected COS cells. Cell surface marker, tetramethylrhodamine concanavalin A (A); EGFP fusion protein (B); co-localization of the two (C).

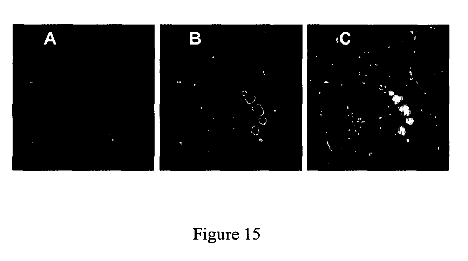

Figure 15 provides confocal microscope images of E4m-EGFP transfected COS cells. Cell surface marker, tetramethylrhodamine concanavalin A (A); EGFP fusion protein (B); co-localization of the two (C).

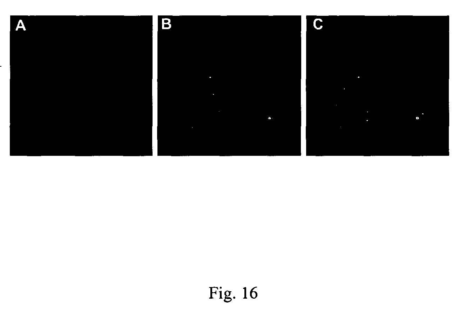

Figure 16 provides confocal microscope images of M231A-E4m-EGFP transfected COS cells. Cell surface marker, tetramethylrhodamine concanavalin A (A); EGFP fusion protein (B); co-localization of the two (C).

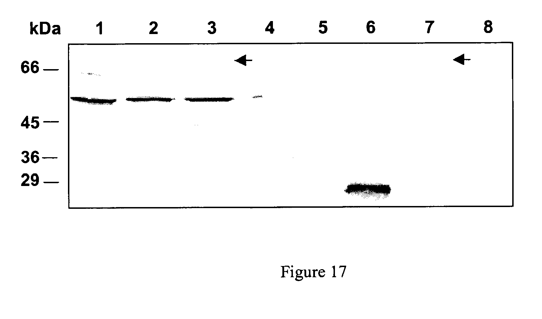

Figure 17 shows expression of EGFP, E4m-EGFP and E5m-EGFP in COS cells. lmmunostaining of EGFP, E4m-EGFP and E5m-EGFP in COS cells identified by Covance Ab PU04 (a polyclonal peptide antibody to the conserved adenine nucleotide binding region of tNOX, lanes 1-4) and anti-GFP antibody (lanes 5-8). Lanes 1 and 5, wild type COS cells. Lanes 2 and 6, pEGFP vector transfected COS cells. Lanes 3 and 7, E4m-EGFP transfected COS cells. Lanes 4 and 8, E5m-EGFP transfected COS cells. The arrows mark the band uniquely expressed in the E4m-EGFP transfected COS cells. There were no detectable bands corresponding to E5m-EGFP.

Figure 18 provides evidence that exon 4 minus antisense (E4AS) and exon 5 minus antisense (E5AS) mediate down-regulation of tNOX mRNA (full length) in transfected HeLa cells. Lipo = Lipofectamine 2000 alone. E4C = Scrambled exon 4 minus antisense control. E5C = Scrambled exon 5 minus antisense control.

Figure 19 is similar to Figure 18 but shows the specific down-regulation of tNOX mRNA (Exon 4 minus) in HeLa cells as detected using E3/5 primers. Lipo = Lipofectamine 2000 alone. E4C = Scrambled exon 4 minus antisense control. E5C = Scrambled exon 5 minus antisense control. E4AS also down-regulates the production of Exon 4 minus tNOX mRNA.

Figure 20 documents that E4AS-mediates down-regulation of tNOX mRNA (Exon 5 minus) in HeLa cells as detected using E4/6 primers. Lipo = Lipofectamine 2000 alone. E4C = Scrambled exon 4 minus antisense as a control. E5C = Scrambled exon 5 minus antisense control. Additionally, E4AS down-regulates the production of Exon 5 minus tNOX mRNA because this mRNA also contains the sense region that is complemented with E4AS.

Figure 21 demonstrates that the growth of HeLa cells transfected with E5AS is no longer inhibited by the tNOX inhibitor epigallocatechin-3-gallate (EGCg). WT = wild type HeLa cells for comparison. Lipo = Lipofectamine 2000 alone. E4C = Scrambled exon 4 minus antisense as a control. E5C = Scrambled exon 5 minus antisense control. Only E5AS targeted to the coding region for E4M mRNA eliminates the ability of the HeLa cells to respond to EGCg, demonstrating that when tNOX mRNA is no longer present (presumably accompanied by a loss of tNOX), the ability of EGCg to inhibit the growth of the cancer cells also is lost.

Figure 22 demonstrates a phenomenon similar to that for Figure 21, except that the tNOX inhibitor is Capsibiol-T.

Figure 23 shows that even in the absence of a tNOX inhibitor, the exon 5 minus antisense (E5AS) which contains the coding region for the Exon 4 minus splice variant mRNA reduces the growth of HeLa cells, as is consistent with the hypothesis that the unregulated growth of these cancer cells is dependent upon tNOX expression.

Full length tNOX mRNA is transcriptionally expressed in human non-cancer cells and tissues as well as in neoplastic cells and tissues. HeLa cells (human cervical carcinoma cell line) express the full length mRNA and the functional 34 kDa tNOX protein. To investigate whether the full length tNOX mRNA is cancer specific, human normal cell lines and tissues were examined by RT-PCR and Northern blot in separate experiments. Commercially available normal human tissue mRNA preparations were probed using the tNOX cDNA sequence in Northern blots. Of the eight different normal human tissues examined, all exhibited actively transcribed full length tNOX mRNA (3.8 Kb as shown in Fig. 2), although the level of transcription in thymus and small intestine was relatively low. Non-cancer cell lines also were examined by RT-PCR to determine if they expressed full length tNOX mRNA. Cancer cell lines HeLa, BT-20, Hek 293, MCF-7, A498 and non-cancer cell lines MCF-10A, WI-38, MCF-12A were analyzed by PCR. The results show that full length tNOX mRNA was transcriptionally expressed in both cancer and non-cancer cell lines.

The full length tNOX cDNA sequence is identical in both cancer cells and non-cancer cells. RT-PCR for both cancer and non-cancer cells was carried out using probes covering the 5'UTR and the 3' end of the tNOX cDNA ORF. The PCR products were cloned into a T7 vector and sequenced. Sequencing results showed no differences in the full length tNOX cDNA sequence between cancer and non-cancer cell lines.

5'-RACE showed there was no upstream ATG beyond that previously found. The tNOX cDNA ORF is 1.83 kb, and it is predicted to encode a protein of 610 amino acids, but there was no naturally expressed 71 kDa tNOX (610 amino acids deduced from tNOX ORF) observed in either cancer or non-cancer cells. The tNOX cDNA sequence has a rare short 22 nucleotide 5'-UTR. Without wishing to be bound by any particular theory, it is believed that this explains why translation does not start from the first ATG. On the other hand, it raises the question whether there is more sequence in the 5'-UTR and/or whether there is another ATG downstream of the current 5' end in cancer cells.

In 5'-RACE, mRNAs of BT-20 and MCF-10A cells were prepared following standard mRNA preparation protocols. The ~240 bp band in the result of 5'-RACE corresponded to exon 1 and exon 2 of tNOX cDNA (Fig. 3). The result showed that the previously determined tNOX cDNA was complete at the 5' end.

Expression of full length tNOX cDNA in non-cancer cells did not result in expression of 34 kDa processed tNOX protein - full-length tNOX cDNA inserted in a mammalian expression vector was expressed in mammalian cells to determine if a 34 kDa tNOX protein was produced. The full length tNOX cDNA was first cloned into the mammalian expression vector pcDNA 3.1. The constructs were then transfected into COS and MCF-10A cells (wild type COS and MCF-10A cells do not express tNOX protein as determined by Western blots and enzymatic assay of drug-response NOX activity). The wild type COS and MCF-10A cells were collected, and the proteins were separated by SDS-PAGE and visualized by Western blotting. A peptide antibody specific for the quinone binding region of tNOX was used in the Western blot.

The results showed that the full-length tNOX cDNA directed expression of a protein of around 71 kDa under the control of a vector promoter in COS and MCF-10A cells. There was no processed form of tNOX protein around 34 kDa observed in the transfectants (Fig. 4). Thus, the 34 kDa processed form of tNOX protein observed on cancer cell membranes and in sera of cancer patients was not processed from the theoretical 71 kDa full length precursor.

To study cancer specific expression of tNOX, MCF-10A and BT-20 plasma membranes were purified. About 0.05 mg samples of plasma membrane proteins of MCF-10A and BT-20 were analyzed by SDS-PAGE (10%) and by Western blot analysis.

For Western blot analysis, proteins were reacted with peptide antibody specific for the tNOX C-terminus containing the conserved adenine nucleotide binding region (KQEMTGVGASLEKRW) (SEQ ID NO:9). Detection was with phosphatase and BCIP and NBT. The 34 kDa protein was present only in BT-20 plasma membranes and not in MCF-10A plasma membranes (Fig. 5).

There were several other bands present in both MCF-10A and BT-20 plasma membranes. Without wishing to be bound by theory, it is believed those bands have sequences homologous to the adenine nucleotide binding region of tNOX that bind the peptide antibody and therefore cross-react with that antibody.

Splice variants of tNOX were found in cancer cells, while fill length tNOX mRNA was present in normal and cancer cells. The tNOX protein is present only on the surface of cancer cells. RT-PCR of MCF-10A, BT-20 and HeLa cells showed an Exon 4 minus form (band 2) and an Exon 5 minus form product (band 3) in both BT-20 and HeLa cells, which are cancer cells, but not in the non-cancer MCF-10A cells (Fig. 6). RT-PCR of MCF-10A, MCF-7, WI-38 and HeLa cells showed an Exon 4 minus form (band 2) and an Exon 5 minus form product (band 3) in both cancer MCF-7 (lane 2) and HeLa cell lines (lane 4) but not in the non-cancer WI-38 (lane 1) and MCF-10A (lane 3) cell lines (Fig.7). The sequences of the E4mtNOX and E5mtNOX cDNAs are given in SEQ ID NO:10 and SEQ ID NO:11, respectively. Exon 4 corresponds to nucleotides 363-571 and exon 5 corresponds to nucleotides 572-805 in SEQ ID NO:12, the full length tNOX cDNA sequence. Several other cancer cell lines and non-cancer cell lines also were tested by RT-PCR. The results showed exon 4 minus form and exon 5 minus form products were present in cancer cell lines and were absent in non-cancer cell lines. Thus, these two alternately spliced variant tNOX messages are uniquely associated with neoplastic cells.

When the NCBI database was searched, two other possible splice variants were found: AK000353 (from a hepatoma cell line) and AL133207 (gene located on chromosome X). Both have the same sequence as exon 2 to exon 8 of tNOX. AK000353 has a 234 bp sequence before exon 2 that is different from that of tNOX. AL133207 has a 348 bp sequence before exon 2 that differs from that of tNOX. Nucleotide 31 to 234 of AK000353 is the same as nucleotide 1 to 204 of AL133207. The presence of these two splice variants were established by RT-RCR (Fig. 8), but they do not appear to be cancer specific.

Additional evidence for a cancer cell-specific expression of exon 4 minus mRNA was provided using exon 4 minus-specific probes generated to the exon 4 minus-specific sequences at the splice junction between exon 3 and exon 5. RNA preparations from HeLa human cervical carcinoma and BT-20 human mammary carcinoma cells clearly contained the expected 670 bp cancer specific product indicative of the absence of exon 4 (Fig. 9). MCF-10A human non-cancer mammary epithelium cells and buffy coats (leukocytes and platelets from a normal volunteer) lacked the PCR product.

Expression of Exon 4 minus and Exon 5 minus tNOX transcripts (E4mtNOX and E5mtNOX, respectively) in COS cells has been studied. The Exon 5 minus cDNA yielded an open reading frame encoding a deduced amino acid sequence of 532 amino acids and a predicted molecular weight of 60.7 kDa (Fig. 10). Unlike the Exon 5 minus DNA, the Exon 4 minus DNA contains in a frame shift that introduces stop codons into the mRNA sequence beginning at nucleotide 592 of the full length sequence. As a result, the next downstream methionine codon now corresponds to M220 of the full length sequence starting at full length sequence nucleotide 680. There is no Kozak sequence at M220. However, initiation at M231 would result in a peptide sequence of 380 amino acids and an apparent molecular weight of 44.2 kDa, which after removal of the signal sequence and further processing (see below) would be expected to generate the functional 34 kDa tNOX protein that is present at the surface of cancer cells. A typical Kozak sequence (A/G)XXATGG) was at nucleotide 710 within exon 5. The putative initiator methionine at nucleotide 713 was followed at L236 by a sequence of 12 hydrophobic residues in the position of a signal sequence and a potential signal peptide cleavage site at nucleotide 788 (A256). The exon 4 minus tNOX transfectants exhibit a 52 kDa band, a 47 kDa band, a 34 kDa band as well as some other processed bands. This is the first time we observed a 34 kDa protein that reacted with the antibody from the expression of a naturally existing mRNA in cancer cells. There are faint reactive 34 kDa bands observed in wild type COS cells and vector only transfectants (Fig. 10). This result showed that the exon 4 minus splice variant mRNA is translated to form a (processed) 34 kDa tNOX protein.

Transport of the 34 kDa tNOX protein to plasma membrane was studied. The functional 34 kDa tNOX protein is on the outer leaflet of cancer cell plasma membranes (7). For the 34 kDa protein expressed from exon 4 minus splice variant is functional, it must reach the plasma membrane. To investigate the sub-cellular localization of the 34 kDa tNOX protein expressed in exon 4 minus COS transfectants, plasma membranes and internal membranes of the transfectants were prepared, the proteins were resolved on SDS-PAGE and then analyzed by Western blotting. Plasma membranes and internal membranes of the exon 4 minus transfectants, along with a whole cell preparation, were probed with a peptide antibody specific for the quinone binding motif of the tNOX protein. The whole cell preparation exhibited 52, 47 and 34 kDa bands. The internal membrane preparation exhibited only 52 and 47 kDa bands. The plasma membrane preparation exhibited only the 34 kDa band. The 52 kDa protein remained in cytoplasm and did not reach the plasma membrane. By contrast, the 34 kDa protein was delivered to the plasma membrane, either as a single product of the exon 4 minus mRNA or a processed form of the 47 or 52 kDa protein (Fig. 11).

Mutation of Met 231 (of SEQ ID NO:13) blocked translational expression of the exon 4 minus splice variant. In the previous overexpression experiments, the 52, 47 and 34 kDa proteins were observed in exon 4 minus splice variant transfectants. The hypothesis to explain the expression of these proteins is that a downstream Met is codon used in the exon 4 minus mRNA during translation (see details about downstream translation initiation below). With our knowledge of the translation initiation and membrane insertion sequence required for the expression of the 34 kDa protein and considering the deletion of exon 4, possible downstream methionine codons include those encoding Met 220, Met 231 and Met314 (With reference to SEQ ID NO:13). Site-directed mutagenesis of the cloned E4m sequence to replace methionine codons with alanine codons (Met220Ala, Met231Ala, and Met314Ala) was carried out. Preliminary results showed that in Met231Ala transfected COS cells, the 47 kDa band was missing (Fig. 12). The size of this band is about the size of protein translated from Met 231. Without wishing to be bound by theory, it is believed that a downstream Met (Met 231) is used as the translation initiation Met in the exon 4 minus mRNA during translation process. This 47 kDa protein is then processed to the 34 kDa active form of tNOX.

The size of the tNOX protein translated in HeLa cell endoplasmic reticulum was determined. HeLa cells were fractionated into endoplasmic reticulum- and plasma membrane-rich fractions by aqueous two-phase partition (17). A recombinant single chain variable region antibody carrying a His tag (partially purified by nickel agarose precipitation) was used to concentrate and identify tNOX related proteins. The endoplasmic reticulum-enriched fraction contained a 47 kDa band absent from the plasma membrane (Fig. 13), whereas the plasma membrane contained on a 34 kDa band.

Confocal microscopy was used to determine the subcellular localization of E4m tNOX-EGFP and full length tNOX-EGFP expressed in COS cells. The tNOX constructs tagged with EGFP at the C terminus were expressed under the control of the cytomegalovirus promoter. Fluorescence microscopy revealed that the E4mtNOX-EGFP fusion protein was localized to plasma membrane. In contrast, full length tNOX-EGFP fusion protein was retained in internal membranes (Figs. 14A-14C, 15A-15C). Mutagenesis of Met231 of E4m-EGFP to Alanine (M231A-E4m-EGFP) was conducted. COS cells transfected with M231A-E4m-EGFP) did not have EGFP fusion protein expressed on the cell surface (Figs. 16A-16C).

EGFP and E4m-EGFP (expressed in COS cells) proteins were analyzed by PAGE and Western blotting using the anti-GFP antibody and peptide Ab (a polyclonal peptide antibody to the conserved adenine nucleotide binding region of tNOX). An approximately 74 kDa band was seen in E4m-EGFP, consistent with the predicted molecular mass of the E4m tNOX (47 kDa) plus EGFP (27 kDa) fusion protein (Fig. 17).

A 34 kDa tNOX protein is found on the surface of cancer cells (1, 2, 8). It is shed into the sera of cancer patients (6, 7) as well as the media of cultured cancer cells (14). A characteristic of the tNOX protein is its drug-response to vanilloids as capsaicin (3) and anti-tumor drugs such as anti-tumor sulfonylureas (15), EGCg (16), and adriamycin (17). It exists in all of the solid cancer tissues, sera of cancer patients, and on the surface of cancer cells (6,7), but not in normal human tissues, sera of healthy volunteers, or non-cancer cells (10). This study was undertaken in part to determine the mechanism for the cancer cell specificity of the 34 kDa cell surface tNOX.

Taken together, the findings indicate that the exon 4 minus splice variant tNOX transcript (E4mtNOX) is unique to neoplastic cells and tissue. First, the exon 4 minus tNOX mRNA exists only in cancer cell lines, as thus far investigated. Second, overexpression experiments show that the exon 4 minus transcript generates the 34 kDa tNOX protein that reacts with a peptide antibody specific for tNOX. In exon 4 minus tNOX cDNA transfected COS cells, at least three proteins (52, 47 and 34 kDa) are expressed that react with the tNOX peptide antibody. The three expressed proteins may be either the result of multiple translation initiation sites, including alternative downstream translation initiation sites, or the 34 kDa protein is a proteolytically processed form originating either from the 52 kDa or 47 kDa protein. The Western blot of transfectants' plasma membranes support the latter hypothesis. The 34 kDa protein is only seen in plasma membranes but not in internal membranes, while the 52 kDa protein exists only in internal membranes and is not found in plasma membranes.

As stated above, there is no evidence of physiological translation of the full length tNOX mRNA despite its widespread transcription. In the cDNA sequence of tNOX, the first ATG starts at nucleotide 23. There are only 22 nucleotides in the 5'UTR. This short 5'UTR makes the first ATG a less likely translation initiation site because of the potential restrictions on ribosome binding.

The scanning mechanism is the current model for translation initiation in eukaryotes (18). After recognition of the cap structure of mRNA, the translation initiation complex forms around 21-24 nucleotides, then begins the scanning for a Met codon. The "first AUG" rule holds for about 90% of the hundreds of eukaryotic mRNAs that have been analyzed (19). Usually the first Met codon starts at about 50-150 nucleotides downstream of the cap (20). In the tNOX sequence, the first Met encoded at nucleotide 23 is believed to be a non-functional initiation sequence for translation. Sequence elements in 5' UTR are involved in regulation of translation (21). The first ATG does not always function as an initiator codon (19, 22). A 5'UTR length is usually smaller than 200 bp because of the limitation of the scanning capacity of the ribosome (19). If the first ATG of full length tNOX mRNA functions as the initiator codon, the 5'UTR of tNOX is only 22 nucleotides, shorter than most functional 5'UTRs (23). Thus it is believed that the initiator ATG is a downstream ATG. A search for a Kozak sequence (A/G)XXAUGG (19) including a downstream initiation site uncovers a Met codon at nucleotide 168 (exon 2), nucleotide 543 (exon 4), nucleotide 713 (exon 5), nucleotide 1155 (exon 8). By deleting an upstream portion of the message through alternative splicing, it may be possible for the scanning ribosome to reach a downstream methionine codon to begin initiation thus to ultimately generate the cell surface 34 kDa form. Another possible explanation is that deletion of exon 4 disrupts the 2nd structure of tNOX mRNA, thus hindering ribosome binding. There may be cancer specific RNA binding proteins involved in this process as well, which increase or decrease the possibility of ribosome binding for downstream translation initiation. Because the processed tNOX protein is on the extracellular side of plasma membrane, the actual translation initiation site must be followed by a membrane insertion sequence. The exon 4 minus splice variant results in a 207 nucleotide deletion. There is no frame shift after the deletion of exon 4. The result is a 68 amino acid deletion and one amino acid change. As discussed above with respect to alternative translation initiation sites, the deletion of exon 4 results in deletion of one Met (at the Met codon beginning at nucleotide 543 of SEQ ID NO:14) in exon 4 and brings the possible downstream Met closer to 5' end. The apparent 52 kDa protein on Western blots may be a processed form that initiates from Met-1 or from a downstream Met. The apparent 34 kDa protein observed on western blots might have come from multiple translation initiation (i.e. starts from a further downstream Met codon), or it could be a processed form from the 52 kDa protein. There are two candidate leader sequences, L236 through S248 and Q323 through H340, with reference to SEQ ID NO:13.

tNOX mRNA synthesis is down-regulated in HeLa cells transfected with antisense oligonucleotides. By using real-time quantitative PCR, E4AS and E5AS were tested for their ability to down-regulate the tNOX mRNA in HeLa cells that were transfected with antisense. As expected, E4AS mediated the down-regulation of full length tNOX mRNA (Fig.18) and Exon 5 minus tNOX mRNA (Fig. 20) because both of them contain the sense region that is complementary to E4AS. E5AS mediated the down-regulation of full length tNOX mRNA (Fig. 18) and Exon 4 minus tNOX mRNA (Fig. 19) because both of them contain the sense region that is complementary to E5AS.

HeLa cells transfected with E5AS were no longer inhibited by EGCg or Capsibiol-T. In vitro cytotoxicity results showed that E5AS transfection could eliminate the inhibition of EGCg (Fig. 21) or Capsibiol-T (Fig. 22) on HeLa cells. In contrast, E4AS transfection has no effect on EGCg (Fig.4) or Capsibiol-T (Fig. 22)'s inhibition on HeLa cells. Since E4AS decreased the full length tNOX mRNA and Exon 5 minus tNOX mRNA levels, it should also decrease the protein level of full length tNOX and Exon 5 minus tNOX. E4AS transfection has no effect on EGCg or Capsibiol-T's inhibition on HeLa cells suggested that full length tNOX and Exon 5 tNOX are not responsible for the EGCg and Capsibiol-T 's function on HeLa cells. E5AS decreased the full length tNOX mRNA and Exon 4 minus tNOX mRNA levels, it should also decrease the protein level of full length tNOX and Exon 4 minus tNOX. E5AS transfection could eliminate the inhibition of EGCg or Capsibiol-T on HeLa cells suggested that full length and/or Exon 4 minus are possible target of EGCg and Capsibiol-T. Because full length tNOX has been ruled out by E4AS transfection, the protein encoded by Exon 4 minus tNOX mRNA is the cellular target of EGCg and Capsibiol-T.

E5AS induced cell death of HeLa cells The effects of antisense oligonucleotides on HeLa cell viability were tested. As show in Fig. 23, E5AS can effectively induced cell death. About 67% cell was killed compared with untreated control. In contrast, the E4AS, E4C control, E5C control and transfectant agent alone had no ability to kill HeLa cells. This result shows that E5AS has sequence-specific toxicity against tumor cells.

The amino acids which occur in the various amino acid sequences referred to in the specification have their usual three- and one-letter abbreviations routinely used in the art: A, Ala, Alanine; C, Cys, Cysteine; D, Asp, Aspartic Acid; E, Glu, Glutamic Acid; F, Phe, Phenylalanine; G, Gly, Glycine; H, His, Histidine; I, Ile, Isoleucine; K, Lys, Lysine; L, Leu, Leucine; M, Met, Methionine; N, Asn, Asparagine; P, Pro, Proline; Q, Gln, Glutamine; R, Arg, Arginine; S, Ser, Serine; T, Thr, Threonine; V, Val, Valine; W, Try, Tryptophan; Y, Tyr, Tyrosine; BCIP, 5-bromo-4-chloro-3-indolyl-phosphate; NBT, nitroblue tetrazolium.

As used herein, neoplasia describes a disease state of a human or an animal in which there are cells and/or tissues which proliferate abnormally. Neoplastic conditions include, but are not limited to, cancers, sarcomas, tumors, leukemias, lymphomas, and the like. The cell surface NADH oxidase/protein disulfide-thiol exchange protein of the present invention is characteristic of neoplastic cells and tissue.

The cell surface marker which is characteristic of diseased cells is described in U.S. Patent No. 5,605,810, issued February 25, 1997, which is incorporated by reference herein, the full length cDNA sequence is presented in WO 01/032673 and in numerous scientific publications of which D. James Morré is sole author or a coauthor. This NADH oxidase/thiol interchange protein is found in the plasma membrane of neoplastic cells and cells infected with viruses, especially retroviruses and protozoan parasites. This neoplasia-specific transcript lacking exon 4, as characterized by the nucleotide sequence set forth in SEQ ID NO:10, is termed E4mtNOX herein (alternatively spliced tumor NADH oxidase, lacking exon 4). The E4mtNOX transcript gives rise to 46 kDa and 44kDa proteins (see Tables 1 and 2 and SEQ ID NO:14 and SEQ ID NO:15). The cell surface 34 kDa protein, processed from a E4mtNOX translation product, is shed into serum and urine in cancer patients, but purification is relatively difficult. Therefore, it was a goal of the present work to obtain a cDNA clone encoding E4m tNOX for use in recombinant production of this protein and for use of the E4mtNOX and E5mtNOX spliced coding sequences or portions thereof in probes and primers for the detection of cancer-specific, alternatively spliced tNOX transcripts. A cDNA for the "full length" message has been described (see, e.g., WO 01/032673 or US 2003-0207340 A1) and its sequence is also available on the website of the National Center for Biotechnology Information, Accession No. AF207881. See also the cDNA sequence set forth in SEQ ID NO:12 herein. The corresponding genomic sequence is available within Accession No. AL049733.

As used herein, neoplasia describes a disease state of a human or an animal in which there are cells and/or tissues which proliferate abnormally. Neoplastic conditions include, but are not limited to, cancers, sarcomas, tumors, leukemias, lymphomas, and the like. The cell surface NADH oxidase/protein disulfide-thiol interchange protein of the present invention characterizes neoplastic cells and tissue as well as certain virus-infected cells (for example, human immunodeficiency virus, feline immunodeficiency virus, etc).

A protein is considered an isolated protein if it is a protein isolated from a host cell in which it is recombinantly produced. It can be purified or it can simply be free of other proteins and biological materials with which it is associated in nature.

An isolated nucleic acid is a nucleic acid the structure of which is not identical to that of any naturally occurring nucleic acid or to that of any fragment of a naturally occurring genomic nucleic acid spanning more than three separate genes. The term therefore covers, for example, (a) a DNA which has the sequence of part of a naturally occurring genomic DNA molecule but is not flanked by both of the coding or noncoding sequences that flank that part of the molecule in the genome of the organism in which it naturally occurs; (b) a nucleic acid incorporated into a vector or into the genomic DNA of a prokaryote or eukaryote in a manner such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, i.e., a gene encoding a fusion protein. Specifically excluded from this definition are nucleic acids present in mixtures of (i) DNA molecules, (ii) transformed or transfected cells, and (iii) cell clones, e.g., as these occur in a DNA library such as a cDNA or genomic DNA library.

As used herein expression directed by a particular sequence is the transcription of an associated downstream sequence. If appropriate and desired for the associated sequence, there the term expression also encompasses translation (protein synthesis) of the transcribed RNA.

In the present context, a promoter is a DNA region which includes sequences sufficient to cause transcription of an associated (downstream) sequence. The promoter may be regulated, i.e., not constitutively acting to cause transcription of the associated sequence. If inducible, there are sequences present which mediate regulation of expression so that the associated sequence is transcribed only when an inducer molecule is present in the medium in or on which the organism is cultivated. In the present context, a transcription regulatory sequence includes a promoter sequence and can further include cis-active sequences for regulated expression of an associated sequence in response to environmental signals.

One DNA portion or sequence is downstream of second DNA portion or sequence when it is located 3' of the second sequence. One DNA portion or sequence is upstream of a second DNA portion or sequence when it is located 5' of that sequence.

One DNA molecule or sequence and another are heterologous to another if the two are not derived from the same ultimate natural source. The sequences may be natural sequences, or at least one sequence can be designed by man, as in the case of a multiple cloning site region. The two sequences can be derived from two different species or one sequence can be produced by chemical synthesis provided that the nucleotide sequence of the synthesized portion was not derived from the same organism as the other sequence.

An isolated or substantially pure nucleic acid molecule or polynucleotide is a polynucleotide which is substantially separated from other polynucleotide sequences which naturally accompany a native transcription regulatory sequence. The term embraces a polynucleotide sequence which has been removed from its naturally occurring environment, and includes recombinant or cloned DNA isolates, chemically synthesized analogues and analogues biologically synthesized by heterologous systems.

A polynucleotide is said to encode a polypeptide if, in its native state or when manipulated by methods known to those skilled in the art, it can be transcribed and/or translated to produce the polypeptide or a fragment thereof. The anti-sense strand of such a polynucleotide is also said to encode the sequence.

A nucleotide sequence is operably linked when it is placed into a functional relationship with another nucleotide sequence. For instance, a promoter is operably linked to a coding sequence if the promoter effects its transcription or expression. Generally, operably linked means that the sequences being linked are contiguous and, where necessary to join two protein coding regions, contiguous and in reading frame. However, it is well known that certain genetic elements, such as enhancers, may be operably linked even at a distance, i.e., even if not contiguous.

The term recombinant polynucleotide refers to a polynucleotide which is made by the combination of two otherwise separated segments of sequence accomplished by the artificial manipulation of isolated segments of polynucleotides by genetic engineering techniques or by chemical synthesis. In so doing one may join together polynucleotide segments of desired functions to generate a desired combination of functions.

Polynucleotide probes include an isolated polynucleotide attached to a label or reporter molecule and may be used to identify and isolate other sequences, for example, those from other mammalian species. Probes comprising synthetic oligonucleotides or other polynucleotides may be derived from naturally occurring or recombinant single or double stranded nucleic acids or be chemically synthesized. Polynucleotide probes may be labeled by any of the methods known in the art, e.g., random hexamer labeling, nick translation, or the Klenow fill-in reaction.

Large amounts of the polynucleotides may be produced by replication in a suitable host cell. Natural or synthetic DNA fragments coding for a protein of interest are incorporated into recombinant polynucleotide constructs, typically DNA constructs, capable of introduction into and replication in a prokaryotic or eukaryotic cell. Usually the construct is suitable for replication in a unicellular host, such as COS cells or a bacterium, but a multicellular eukaryotic host may also be appropriate, with or without integration within the genome of the host cell. Commonly used prokaryotic hosts include strains of Escherichia coli, although other prokaryotes, such as Bacillus subtilis or a pseudomonad, may also be used. Eukaryotic host cells include yeast, fungi, plant, insect, amphibian and avian species, but the regulated expression of a protein of interest a mammalian cell is preferred. Such factors as ease of manipulation, ability to appropriately glycosylate expressed proteins, degree and control of protein expression, ease of purification of expressed proteins away from cellular contaminants or other factors influence the choice of the host cell.

The polynucleotides may also be produced by chemical synthesis, e.g., by the phosphoramidite method described by Beaucage and Caruthers (1981) Tetra. Letts. 22: 1859-1862 or the triester method according to Matteuci et al. (1981) J. Am. Chem. Soc. 103: 3185, and may be performed on commercial automated oligonucleotide synthesizers. A double-stranded fragment may be obtained from the single stranded product of chemical synthesis either by synthesizing the complementary strand and annealing the strand together under appropriate conditions or by adding the complementary strand using DNA polymerase with an appropriate primer sequence.

DNA constructs prepared for introduction into a prokaryotic or eukaryotic host will typically comprise a replication system (i.e. vector) recognized by the host, including the intended DNA fragment encoding the desired polypeptide, and will preferably also include transcription and translational initiation regulatory sequences operably linked to the polypeptide-encoding segment. Expression systems (expression vectors) may include, for example, an origin of replication or autonomously replicating sequence (ARS) and expression control sequences, a promoter, an enhancer and necessary processing information sites, such as ribosome-binding sites, RNA splice sites, polyadenylation sites, transcriptional terminator sequences, and mRNA stabilizing sequences. Signal peptides may also be included where appropriate from secreted polypeptides of the same or related species, which allow the protein to cross and/or lodge in cell membranes or be secreted from the cell.

An appropriate promoter and other necessary vector sequences will be selected so as to be functional in the host. Examples of workable combinations of cell lines and expression vectors are described in Sambrook et al. (1989) vide infra; Ausubel et al. (Eds.) (1995) Current Protocols in Molecular Biology, Greene Publishing and Wiley Interscience, New York; and Metzger et al. (1988) Nature, 334: 31-36. Many useful vectors for expression in bacteria, yeast, fungal, mammalian, insect, plant or other cells are well known in the art and may be obtained such vendors as Invitrogen, Stratagene, New England Biolabs, Promega Corporation, and others. In addition, the construct may be joined to an amplifiable gene (e.g., DHFR) so that multiple copies of the gene may be made. For appropriate enhancer and other expression control sequences, see also Enhancers and Eukaryotic Gene Expression, Cold Spring Harbor Press, N.Y. (1983). While such expression vectors may replicate autonomously, they may less preferably replicate by being inserted into the genome of the host cell.

Expression and cloning vectors will likely contain a selectable marker, that is, a gene encoding a protein necessary for the survival or growth of a host cell transformed with the vector. Although such a marker gene may be carried on another polynucleotide sequence co-introduced into the host cell, it is most often contained on the cloning vector. Only those host cells into which the marker gene has been introduced will survive and/or grow under selective conditions. Typical selection genes encode proteins that confer resistance to antibiotics or other toxic substances, e.g., ampicillin, neomycin, methotrexate, etc.; complement auxotrophic deficiencies; or supply critical nutrients not available from complex media. The choice of the proper selectable marker will depend on the host cell; appropriate markers for different hosts are known in the art.

Recombinant host cells, in the present context, are those which have been genetically modified to contain an isolated DNA molecule of the instant invention. The DNA can be introduced by any means known to the art which is appropriate for the particular type of cell, including without limitation, transformation, lipofection or electroporation.

It is recognized by those skilled in the art that the DNA sequences may vary due to the degeneracy of the genetic code and codon usage. All DNA sequences which code for the polypeptide or protein of interest are included in this invention.

Additionally, it will be recognized by those skilled in the art that allelic variations may occur in the DNA sequences which will not significantly change activity of the amino acid sequences of the peptides which the DNA sequences encode. All such equivalent DNA sequences are included within the scope of this invention and the definition of the regulated promoter region. The skilled artisan will understand that the sequence of the exemplified sequence can be used to identify and isolate additional, nonexemplified nucleotide sequences which are functionally equivalent to the sequences given.

Hybridization procedures are useful for identifying polynucleotides with sufficient homology to the subject regulatory sequences to be useful as taught herein. The particular hybridization technique is not essential to the subject invention. As improvements are made in hybridization techniques, they can be readily applied by one of ordinary skill in the art.

A probe and sample are combined in a hybridization buffer solution and held at an appropriate temperature until annealing occurs. Thereafter, the membrane is washed free of extraneous materials, leaving the sample and bound probe molecules typically detected and quantified by autoradiography and/or liquid scintillation counting. As is well known in the art, if the probe molecule and nucleic acid sample hybridize by forming a strong non-covalent bond between the two molecules, it can be reasonably assumed that the probe and sample are essentially identical, or completely complementary if the annealing and washing steps are carried out under conditions of high stringency. The probe's detectable label provides a means for determining whether hybridization has occurred.

In the use of the oligonucleotides or polynucleotides as probes, the particular probe is labeled with any suitable label known to those skilled in the art, including radioactive and non-radioactive labels. Typical radioactive labels include ³²P, ³⁵S, and the like. Non-radioactive labels include, for example, ligands such as biotin or thyroxine, as well as enzymes such as hydrolases or peroxidases, or a chemiluminescer such as luciferin, or fluorescent compounds like fluorescein and its derivatives. Alternatively, the probes can be made inherently fluorescent as described in International Application No. WO 93/16094.

Various degrees of stringency of hybridization can be employed. The more stringent the conditions, the greater the complementarity that is required for duplex formation. Stringency can be controlled by temperature, probe concentration, probe length, ionic strength, time, and the like. Preferably, hybridization is conducted under moderate to high stringency conditions by techniques well know in the art, as described, for example in Keller, G.H., M.M. Manak (1987) DNA Probes, Stockton Press, New York, NY., pp. 169-170, hereby incorporated by reference.

As used herein, moderate to high stringency conditions for hybridization are conditions which achieve the same, or about the same, degree of specificity of hybridization as the conditions employed by the current inventors. An example of high stringency conditions are hybridizing at 68° C in 5X SSC/5X Denhardt's solution/0.1% SDS, and washing in 0.2X SSC/0.1% SDS at room temperature. An example of conditions of moderate stringency are hybridizing at 68° C in 5X SSC/5X Denhardt's solution/0.1% SDS and washing at 42° C in 3X SSC. The parameters of temperature and salt concentration can be varied to achieve the desired level of sequence identity between probe and target nucleic acid. See, e.g., Sambrook et al. (1989) vide infra or Ausubel et al. (1995) Current Protocols in Molecular Biology, John Wiley & Sons, NY, NY, for further guidance on hybridization conditions.

Specifically, hybridization of immobilized DNA in Southern blots with ³²P-labeled gene specific probes was performed by standard methods (Maniatis et al.) In general, hybridization and subsequent washes were carried out under moderate to high stringency conditions that allowed for detection of target sequences with homology to the exemplified sequences. For double-stranded DNA gene probes, hybridization can be carried out overnight at 20-25°C below the melting temperature (Tm) of the DNA hybrid in 6X SSPE 5X Denhardt's solution, 0.1% SDS, 0.1 mg/ml denatured DNA. The melting temperature is described by the following formula (Beltz, G.A., Jacobe, T.H., Rickbush, P.T., Chorbas, and F.C. Kafatos (1983) Methods of Enzymology, R. Wu, L, Grossman and K. Moldave (eds) Academic Press, New York 100:266-285).

Tm=81.5° C + 16.6 Log[Na+]+0.41(+G+C)-0.61(%formamide)-600/length of duplex in base pairs.

Washes are typically carried out as follows: twice at room temperature for 15 minutes in 1X SSPE, 0.1% SDS (low stringency wash), and once at TM-20° C for 15 minutes in 0.2X SSPE, 0.1% SDS (moderate stringency wash).

For oligonucleotide probes, hybridization was carried out overnight at 10-20° C below the melting temperature (Tm) of the hybrid 6X SSPE, 5X Denhardt's solution, 0.1% SDS, 0.1 mg/ml denatured DNA. Tm for oligonucleotide probes was determined by the following formula: TM(°C)=2(number T/A base pairs +4(number G/C base pairs) (Suggs, S.V., T. Miyake, E.H., Kawashime, M.J. Johnson, K. Itakura, and R.B. Wallace (1981) ICB-UCLA Symp. Dev. Biol. Using Purified Genes, D.D. Brown (ed.), Academic Press, New York, 23:683-693).

Washes were typically carried out as follows: twice at room temperature for 15 minutes 1X SSPE, 0.1% SDS (low stringency wash), and once at the hybridization temperature for 15 minutes in 1X SSPE, 0.1% SDS (moderate stringency wash).

In general, salt and/or temperature can be altered to change stringency. With a labeled DNA fragment of more than 70 or so bases in length, the following conditions can be used: Low, 1 or 2X SSPE, room temperature; Low, 1 or 2X SSPE, 42°C; Moderate, 0.2X or 1X SSPE, 65° C; and High, 0.1X SSPE, 65° C.

Duplex formation and stability depend on substantial complementarity between the two strands of a hybrid, and, as noted above, a certain degree of mismatch can be tolerated. Therefore, the probe sequences of the subject invention include mutations (both single and multiple), deletions, insertions of the described sequences, and combinations thereof, wherein said mutations, insertions and deletions permit formation of stable hybrids with the target polynucleotide of interest. Mutations, insertions, and deletions can be produced in a given polynucleotide sequence in many ways, and those methods are known to an ordinarily skilled artisan. Other methods may become known in the future.

Mutational, insertional, and deletional variants of the disclosed nucleotide sequences can be readily prepared by methods which are well known to those skilled in the art. These variants can be used in the same manner as the exemplified primer sequences so long as the variants have substantial sequence homology with the original sequence. As used herein, substantial sequence homology refers to homology which is sufficient to enable the variant polynucleotide to function in the same capacity as the polynucleotide from which the probe was derived. Preferably, this homology is greater than 80%, more preferably, this homology is greater than 85%, even more preferably this homology is greater than 90%, and most preferably, this homology is greater than 95%. The degree of homology or identity needed for the variant to function in its intended capacity depends upon the intended use of the sequence. It is well within the skill of a person trained in this art to make mutational, insertional, and deletional mutations which are equivalent in function or are designed to improve the function of the sequence or otherwise provide a methodological advantage.

Polymerase Chain Reaction (PCR) is a repetitive, enzymatic, primed synthesis of a nucleic acid sequence. This procedure is well known and commonly used by those skilled in this art (see Mullis, U.S. Patent Nos. 4,683,195, 4,683,202, and 4,800,159; Saiki et al. (1985) Science 230:1350-1354 and commercial suppliers of reagents for PCR, real time PCR and reverse transcriptase PCR). In the context of the present application, RT-PCR is reverse transcriptase PCR. PCR is based on the enzymatic amplification of a DNA fragment of interest that is flanked by two oligonucleotide primers that hybridize to opposite strands of the target sequence. The primers are oriented with the 3' ends pointing towards each other. Repeated cycles of heat denaturation of the template, annealing of the primers to their complementary sequences, and extension of the annealed primers with a DNA polymerase result in the amplification of the segment defined by the 5' ends of the PCR primers. Since the extension product of each primer can serve as a template for the other primer, each cycle essentially doubles the amount of DNA template produced in the previous cycle. This results in the exponential accumulation of the specific target fragment, up to several million-fold in a few hours. By using a thermostable DNA polymerase such as the Taq polymerase, which is isolated from the thermophilic bacterium *Thermus aquaticus,* the amplification process can be completely automated. Other enzymes which can be used are known to those skilled in the art.

It is well known in the art that the polynucleotide sequences of the present invention can be truncated and/or mutated such that certain of the resulting fragments and/or mutants of the original full-length sequence can retain the desired characteristics of the full-length sequence. A wide variety of restriction enzymes which are suitable for generating. fragments from larger nucleic acid molecules are well known. In addition, it is well known that an exonuclease such as *Bal*31 can be conveniently used for time-controlled limited digestion of DNA. See, for example, Maniatis (1982) Molecular Cloning: A Laboratory Manual, Cold Spring Harbor Laboratory, New York, pages 135-139, incorporated herein by reference. See also Wei et al. (1983 J. Biol. Chem. 258:13006-13512. By use of *Ba*/31 exonuclease, the ordinarily skilled artisan can remove nucleotides from either or both ends of the subject nucleic acids to generate a wide spectrum of fragments which are functionally equivalent to the subject nucleotide sequences. One of ordinary skill in the art can, in this manner, generate hundreds of fragments of controlled, varying lengths from locations all along the original molecule. The ordinarily skilled artisan can routinely test or screen the generated fragments for their characteristics and determine the utility of the fragments as taught herein. It is also well known that the mutant sequences of the full length sequence, or fragments thereof, can be easily produced with site directed mutagenesis. See, for example, Larionov, O.A. and Nikiforov, V.G. (1982) Genetika 18(3):349-59; Shortle, D, DiMaio, D., and Nathans, D. (1981) Annu. Rev. Genet. 15:265-94; both incorporated herein by reference.

As used herein percent sequence identity of two nucleic acids is determined using the algorithm of Karlin and Altschul (1990) Proc. Natl. Acad. Sci. USA 87:2264-2268, modified as in Karlin and Altschul (1993) Proc. Natl. Acad. Sci. USA 90:5873-5877. Such an algorithm is incorporated into the NBLAST and XBLAST programs of Altschul et al. (1990) J. Mol. Biol. 215:402-410. BLAST nucleotide searches are performed with the NBLAST program, score = 100, word length = 12, to obtain nucleotide sequences with the desired percent sequence identity. To obtain gapped alignments for comparison purposes, Gapped BLAST is used as described in Altschul et al. (1997) Nucl. Acids. Res. 25:3389-3402. When utilizing BLAST and Gapped BLAST programs, the default parameters of the respective programs (NBLAST and XBLAST) are used. See, for example, the National Center for Biotechnology Information website on the internet.

Monoclonal or polyclonal antibodies, preferably monoclonal, specifically reacting with an E4mtNOX-derived protein may be made by methods known in the art. See, e.g., Harlow and Lane (1988) Antibodies: A Laboratory Manual, Cold Spring Harbor Laboratories; Goding (1986) Monoclonal Antibodies: Principles and Practice, 2d ed., Academic Press, New York.

Standard techniques for cloning, DNA isolation, amplification and purification, for enzymatic reactions involving DNA ligase, DNA polymerase, restriction endonucleases and the like, and various separation techniques are those known and commonly employed by those skilled in the art. A number of standard techniques are described in Sambrook et al. (1989) Molecular Cloning, Second Edition, Cold Spring Harbor Laboratory, Plainview, New York; Maniatis et al. (1982) Molecular Cloning, Cold Spring Harbor Laboratory, Plainview, New York; Wu (ed.) (1993) Meth. Enzymol. 218, Part I; Wu (ed.) (1979) Meth Enzymol. 68; Wu et al. (eds.) (1983) Meth. Enzymol. 100 and 101; Grossman and Moldave (eds.) Meth. Enzymol. 65; Miller (ed.) (1972) Experiments in Molecular Genetics, Cold Spring Harbor Laboratory, Cold Spring Harbor, New York; Old and Primrose (1981) Principles of Gene Manipulation, University of California Press, Berkeley; Schleif and Wensink (1982) Practical Methods in Molecular Biology*;* Glover (ed.) (1985) DNA Cloning Vol. I and II, IRL Press, Oxford, UK; Hames and Higgins (eds.) (1985) Nucleic Acid Hybridization, IRL Press, Oxford, UK; and Setlow and Hollaender (1979) Genetic Engineering: Principles and Methods, Vols. 1-4, Plenum Press, New York. Abbreviations and nomenclature, where employed, are deemed standard in the field and commonly used in professional journals such as those cited herein.

Although the description herein contains certain specific examples and statements, these should not be construed as limiting the scope of the invention but as merely providing illustrations of some embodiments of the invention. For example, thus the scope of the invention should be determined by the appended claims and their equivalents, rather than by the examples given. Any variations in the exemplified articles which occur to the skilled artisan are intended to fall within the scope of the present invention.

### EXAMPLES

### Example 1: Materials

Most chemicals and reagents were from Sigma-Aldrich, St. Louis, MO. DNA markers, Taq polymerase, and Random Hexamer Priming Kit were from Promega Corporation Madison, WI. SuperScript II Reverse Transcriptase was from GIBCO/Invitrogen, Carlsbad, CA. Restriction enzymes were from New England Biolabs, Beverly, MA. Human Multiple Tissue Northern Blot was from Clontech, Palo Alto, CA. Fast-Link DNA Ligation Kit was from Epicentre, Madison, WI. Cloned pfu DNA polymerase and *E. coli* strain XL1-blue were from Stratagene, La Jolla, CA. Gel Purification kit, Plasmid DNA Mini and Midi Purification Kit were from Qiagen, Valencia, CA. Mammalian expression vector pcDNA 3.1, and Calcium Phosphate Transfection Kit were from Invitrogen, Carlsbad, CA. Alkaline phosphatase conjugated monoclonal anti-rabbit IgG was from Sigma Chemical Co., St. Louis, MO. Peptide antibodies PU02 and PU04 were generated by Covance Research Products, Inc., Denver, PA.

### Example 2: Total RNA preparation

Approximately 10⁶ cells of HeLa (human cervical carcinoma), BT-20 (human breast adenocarcinoma), and MCF-1OA (human breast epithelial cells) were collected and washed twice with cold PBS, pH 7.4. Each cell pellet were resuspended in 300 µl RNA Preparation Solution I (10 mM Tris-Cl, 0.15 M NaCl, 1.5 M MgCl₂, 0.65% NP-40, DEPC treated). After centrifugation at 800 g for 10 min, the upper phase was transferred to a new tube and 200 µl RNA Preparation Solution II (7 M urea, 1% SDS, 0.35 M NaCl, 10 mM EDTA, 10 mM Tris-Cl, DEPC treated) were added. Then 400 µl phenol/ chloroform/isoamyl alcohol (50:50:1) were added and the mixture was centrifuged at 15,000 g for 5 min. The clear phase that contained RNA was precipitated with 95% ethanol and dissolved in DEPC (diethyl pyrocarbonate) treated water.

### Example 3: RT-PCR

A 20 µl reaction volume containing 1 µl Oligo (dT)₁₂₋₁₈ (500 µg/ml), 1 µl 10 mM dNTP, 4 µl 5 X First-Strand Buffer, 2 µl 0.1 M DTT, 5 µg of total RNA, and 1 µl Reverse Transcriptase was used. Reaction mixture was incubated at 42 °C for 1 hour, followed by heating at 70 °C for 15 min to inactivate. The synthesized first-strand cDNA was amplified by PCR using primers 5'-CAGCCGATAACAGTAGAACTCTGA-3' (forward) (SEQ ID NO:1) and 5'- CTGATTCCTCAGTTTCTTTTGTTTCTG-3' (reverse) (SEQ ID NO:2). PCR products were separated on a 1% agarose gel and recovered a using Gel Extraction Kit(Qiagen). Purified PCR products were sequenced to verify identities.

### Example 4: Northern Blot

cDNA probes used in the northern blot experiments were generated from PCR using a pcDNA 3.1-tNOX plasmid. The probes were labeled with (α-³²P) dCTP using Random Hexamer Priming Kit (Promega) prior to use. Two probes were used: 1.2 kb tNOX cDNA corresponding to tNOX sequence 680 bp to 1,830 bp; and exon 4 of tNOX. The human MTN (Multiple Tissue Northern) blot was incubated with prehybridization solution at 65 °C for 6 hours. Then hybridization solution with either of the probes was added and incubated at 65 °C overnight. The MTN Blot was washed and autoradiograms (X ray film) were exposed at -80 °C.

### Example 5: 5'- Rapid Amplification of cDNA End (5'-RACE)

Total RNA from BT-20 and MCF-10A cells were prepared by the guanidine isothiocyanate/acid-phenol method. Total RNA was quantified by Hitachi U-1100 UV spectrometry and stored at -20°C in 30 µl DEPC treated water. A custom primer, 109L, is a 20 nt reverse primer matching the end of exon 2: 5'-GGTAAAATTGGTGTCCGGC-3' (SEQ ID NO:3), was used in first strand synthesis and the later in the PCR process. 20 mM Tris-HC1 (pH 8.4), 50 mM KC1, 2.5 mM MgCl₂, 10 mM DTT, 100 nM cDNA custom primer 109L, 400 µM each dATP, dCTP, dGTP, dTTP, 5 µg RNA, and 200 units of Superscript II RT were used in the first strand cDNA synthesis. After incubation at 42° C for one hour, the reaction was terminated by incubation at 70° C for 15 min. Then RNAse H was added to the mix to digest the RNA strand in RNA/cDNA hetero-double strand. The digest was transferred to the GlassMAX spin cartridge and centrifuge at 13,000 g for 20 s. After three washes, the first strand cDNA was purified and concentrated for the next step of TdT tailing. TdT adds several Cs at the 3' end of the first strand cDNA. Using anchoring primers and the customer primer 109L, the cDNA was specifically amplified and resolved on 1 % agarose gel. It was further cloned into T-vector and sequenced.

### Example 6: RT-PCR to detect exon 4 minus tNOX mRNA in cancer and non-cancer cells

Total RNA was prepared from 2 X 10⁷ cells. The forward primer, a 20 mer oligonucleotide (TCCCTCCAAATCCAATACCG) (SEQ ID NO:4) was from the junction of exon 3 and exon 5 with 15 nucleotides from exon 3 and 5 nucleotides from exon 5. The reverse primer (CTGATTCCTCAGTTTCTTTTCTTTCTG) (SEQ ID NO:5) was from the end of exon 8:. A product of 670 nucleotides was expected. Glyceraldehyde-3-phosphate dehydrogenase (GAPDH) primers were used as controls (640 nucleotides).

### Example 7: Expression of E4mtNOX and E5mtNOX messages in COS cells

Parts of the Exon 4 minus and Exon 5 minus forms of tNOX (E4mtNOX and E5m tNOX) cDNAs were first amplified by PCR using primers 5'-CCGATAACAGTAGAACTCTGAAC-3' (forward) (SEQ ID NO:6) and 5'-GGCTGATTCCTCAGTTTCTTTTGTTTC-3' (reverse) (SEQ ID NO:7). The PCR product was then ligated to pGEM-T Easy Vector (Promega, Madison, WI) for sequencing. The construct was digested with Notl. The digested products were separated on an agarose gel and extracted using a DNA Extraction Kit (Qiagen, Valencia, CA). The DNA then was double digested with Banl and Asel. The digested products were also separated on an agarose gel and extracted (Fragment A). The pcDNA3.1-full length tNOX was double digested with HindIII and BamHI and a fragment containing pcDNA3.1 and part of tNOX (Fragment D) and the other fragment F was obtained. Fragment F was then double digested with Banl and Asel to get 3 fragments. There was a fragment between HindIII and Banl (Fragment B), a fragment between Banl and Asel (Fragment G) and a fragment between Asel and BamHI (Fragment C). All of these digested products were separated on an agarose gel and extracted. Finally, 4 fragments (Fragments A, B, C, and D) were ligated using a Fast-link kit (Epicenter). The resulting plasmid was used to transfect COS cells.

DNA sequences of the ligation products were confirmed by sequencing. Expression of the E4mtNOX and E5mtNOX protein was confirmed by SDS-PAGE with immunoblotting. Immunoblot analysis was with tNOX peptide antibody. Detection used alkaline phosphate-conjugated anti-rabbit antibody. The E4mtNOX and E5tNOX cDNA sequences are given in SEQ ID NO:10 and SEQ ID NO:11, respectively, while the full length tNOX cDNA sequence is depicted in SEQ ID NO:12.

COS cells (SV-40 transformed African monkey kidney cell line) were plated one day prior to transfection at 4 × 10⁵ cells per 100 mm dish. 36 µl of 2 M CaCl₂ and 30 µg of pcDNA3.1-E5m tNOX in 300 µl sterile H₂O were slowly added dropwise into 300 µl of 2 X Hepes Buffered Saline (HBS) at room temperature for 30 min. The transfection mixtures then were added dropwise to the media to the cells and incubated overnight at 37°C. After overnight exposure to the DNA precipitate, the cells were washed twice with PBS and 3 ml of DMSO were added for 2.5 min. The DMSO then was removed and cells were fed with fresh media for 2-3 days. For selection of stable transfectants, 0.5 mg/ml of G418 sulfate (Invitrogen) was added into the media twice a week and the cultures were maintained until colonies 2-3 mm in diameter were formed. A total of 4 colonies were selected and trypsinized individually followed by transferring into wells of a 24-well plate and then into a 35 mm petri dish. Cells were harvested at 80% confluency.

### Example 8: Site-Directed Mutagenesis

Codons encoding methionines M220, M231, and M314 were replaced by alanine codons by site-directed mutagenesis according to Braman et al. (13).

### Example 9: Western Blot

Approximately 10⁶ COS cells were transfected with pcDNA 3.1, pcDNA 3.1-tNOX, or pcDNA 3.1- exon 4 minus tNOX. Whole cell extracts were dissolved in 100 µl 10 mM Tris-Cl and immediately loaded into SDS-PAGE gels. After transfer, the nitrocellulose membrane was incubated with either PU02 or PU04 anti-tNOX antibody at 4 °C overnight. PU02 is polyclonal peptide antibody to the tNOX c-terminal containing the putative adenine nucleotide binding motif (T589GVGASLEKRWKFCGFE605) (SEQ ID NO:8) PU04 is polyclonal peptide antibody to the tNOX putative quinone binding site which begins at amino acid 394, with reference to SEQ ID NO:13 (EEMTECRREEEMEMSDDEIEEMTETK) (SEQ ID NO:9). The membrane was incubated with alkaline phosphatase conjugated monoclonal anti-rabbit IgG at room temperature for one h.

### Example 10: Construction of Plasmid pE4m-EGFP

A pair of oligodeoxyribonucleotide primers (5'-GATCTCGAGCTCAAGCTTGACCACACAATGCAA-3', SEQ ID NO:16, forward; and 5'-ATCCCGGGCCCGCGGTACCGTCAGCTTCAAGCC-3', SEQ ID NO:17, reverse) were used to amplify the E4mtNOX coding region by PCR. The PCR products were digested with HindIII and KpnI restriction endonucleases and the DNA fragment was purified by agarose gel electrophoresis. The plasmid pEGFP-NI was digested with HindIII and Kpnl, and the backbone of the expression plasmid was gel-isolated. The two purified fragments were then joined to give pE4m-EGFP.

### Example 11: Expression of EGFP-Fused E4mtNOX in COS cells

The E4m-EGFP expression plasmid was constructed using pEGFP-N1, which allow expression of the enhanced green fluorescent protein fused at the C-terminus of E4m tNOX. *E. coli* cells were transformed with pE4m-EGFP. The plasmid was isolated from transformed *E. coli* cells. COS cells were transfected with pE4m-EGFP using calcium phosphate (Stratagene, Inc.) transfection and DMSO shock according to the instructions provided by the supplier.

### Example 12: Confocal Laser Scanning Microscopy

Cells were grown in corner glass chambers and observed on a Bio-Rad MRC-1024 confocal microscope. Fluorescence of EGFP was excited using a 488 nm krypton/argon laser, and emitted fluorescence was detected with 515-540 band pass filter. For tetramethylrhodamine concanavalin A, a 568 nm krypton/argon laser was used for excitation and fluorescence was detected with a 590 nm band pass filter. Cells were rinsed with cold PBS and treated with tetramethylrhodamine concanavalin A (10 µg/ml in PBS) for 2 min at 4° C. After a final rinse with cold PBS, the cells were observed with a confocal microscope using the 60 X oil immersion lens.

### Example 13. Antisense Oligonucleotides Synthesis

E4AS, E5AS and their corresponding scrambled controls E4C and E5C were synthesized. There were 3 phosphorothioate linkages at each end of the oligonucleotides. Each final product was desalted and purified by HPLC (IDT Inc). Their sequences are shown in Table 5.

### Example 14. Real-Time Quantitative PCR.

Total RNA from HeLa cells treated with antisense oligonucleotides for 6 h was isolated using RNeasy mini kits (Qiagen). Specific tNOX mRNA was measured using a real-time quantitative PCR method. Sequences of primers and their nucleotide position on tNOX are shown in Table 6. Primer FLS and FLR was used to detect full length tNOX mRNA. Primer E3/5 S and E3/5 R was used to detect E4m tNOX mRNA. Primer E4/6 S and E4/6 R was used to detect E5m tNOX mRNA. The RNA was reverse-transcribed using M-MLV reverse transcriptase (Promega Corporation, Madison, WI). Real-time PCR was performed using the SYBR Green Supermix (BioRad Laboratories, Hercules, CA). Real-time PCR was 45 cycles. tNOX mRNA level of each sample was calculated relative to Lipofectamine 2000 (Invitrogen Corporation, Carlsbad, CA) transfection control cells. tNOX mRNA levels were determined by the cycle threshold method and were normalized for GAPDH mRNA content.

### Example 15. Treatment of Cultured HeLa cells with Antisense Oligonucleotides and EGGg, Capsibiol-T.

One day before in vitro transfection, HeLa cells were plated in 10 cm tissue culture plates. Antisense oligonucleotides were delivered into cells with the Lipofectamine 2000 transfection reagent. For 96-well plates, transfection complex was added then cells were seeded according to the manufacturer's protocol. On 24 h transfection, EGCg (1 mM) and Capsibiol-T (1.25 mg/ml) were added, and cells were incubated for an additional 48 h before analysis. Example 16. In Vitro Cytotoxicity Assay

Cells were transfected with oligonucleotides using the Lipofectamine 2000 transfection reagent for 24 h, and then they were incubated for 48 h at 37°C in the presence or absence of EGCg. At the end of the incubation period, the media were decanted, and the cells washed twice with cold PBS. The cells were then fixed in 2.5% (v/v) glutaraldehyde for 0.5 h and washed with distilled water. Viable cells were stained with 200 µl of 0.5% aqueous crystal violet solution for 0.5 h and then washed exhaustively with distilled water. Acetic acid (200 µl; 33% (v/v)) was added to solubilize the cells for 0.5 h, and the absorbance read at 580 nm using plate reader (Lin et al., 1996). The percentage of surviving cells treated with EGCg was normalized to untreated controls according to the following formula: [(b - c) × 100]/(a - c), where a, b, and c are the absorbance values of cells in medium without EGCg, cells in medium with EGCg, and medium alone, as background, respectively.

### Example 17. Statistical Analyses

The Student's t test was used to measure statistical significance between two treatment groups. Data were considered significant for a P<0.05.

The amino acid sequences of the approximately 46 kDa and approximately 44 kDa proteins, which are expression products of the E4mtNOX mRNA, are given in Tables 1 and 2, respectively. See also, SEQ ID NO:14 and SEQ ID NO:15.

**Table 1. Amino Acid Sequence of 46 kDa Translation Product of E4mtNOX mRNA. See also SEQ ID NO:14 (protein) and SEQ ID NO:10 (cDNA)**

| 391 AA; 45695 MW; |
|---|
| MLAREERHRR RMEEERLRPP SPPPVVHYSD HECSIVAEKL KDDSKFSEAV QTLLTWIERG |
| EVNRRSANNF YSMIQSANSH VRRLVNEKAA HEKDMEEAKE KFKQALSGIL IQFEQIVAVY |
| HSASKQKAWS HFTKAQRKNI SVWCKQAEEI RNIHNDELMG IRREEEMEMS DDEIEEMTET |
| KETEESALVS QAEALKEEND SLRWQLDAYR NEVELLKQEQ GKVHREDDPN KEQQLKLLQQ |
| ALQGMQQHLL KVQEEYKKE AELEKLKDDK LQVEKMLENL KEKESCASRL CASNQDSEYP |
| LEKTMNSSPI KSEREALLVG IISTFLHVHP FGASIEYICS YLHRLDNKIC TSDVECLMGR |
| LQHTFKQEMT GVGASLEKRW KFCGFEGLKL T |

**Table 2. Amino Acid Sequence of 44 kDa Translation Product. See also SEQ ID NO:15.**

| SEQUENCE 380 AA; 44203 MW; |
|---|
| MEEERLRPPS PPPVVHYSDH ECSIVAEKLK DDSKFSEAVQ TLLTWIERGE VNRRSANNFY |
| SMIQSANSHV RRLVNEKAAH EKDMEEAKEK FKQALSGILI QFEQIVAVYH SASKQKAWDH |
| FTKAQRKNIS VWCKQAEEIR NIHN DELMGI RREEEMEMSD DEIEEMTETK ETEESALVSQ |
| AEALKEENDS LRWQLDAYRN EVELLKQEQG KVHREDDPNK EQQKLLQQA LQGMQQHLLK |
| VQEEYKKEA ELEKLKDDKL QVEKMLENLK EKESCASRLC ASNQDSEYPL EKTMNSSPIK |
| SEREALLVGI ISTFLHVHPF GASIEYICSY LHRLDNKICT SDVECLMGRL QHFKQEMTG |
| VGASLEKRWK FCGFEGLKLT |

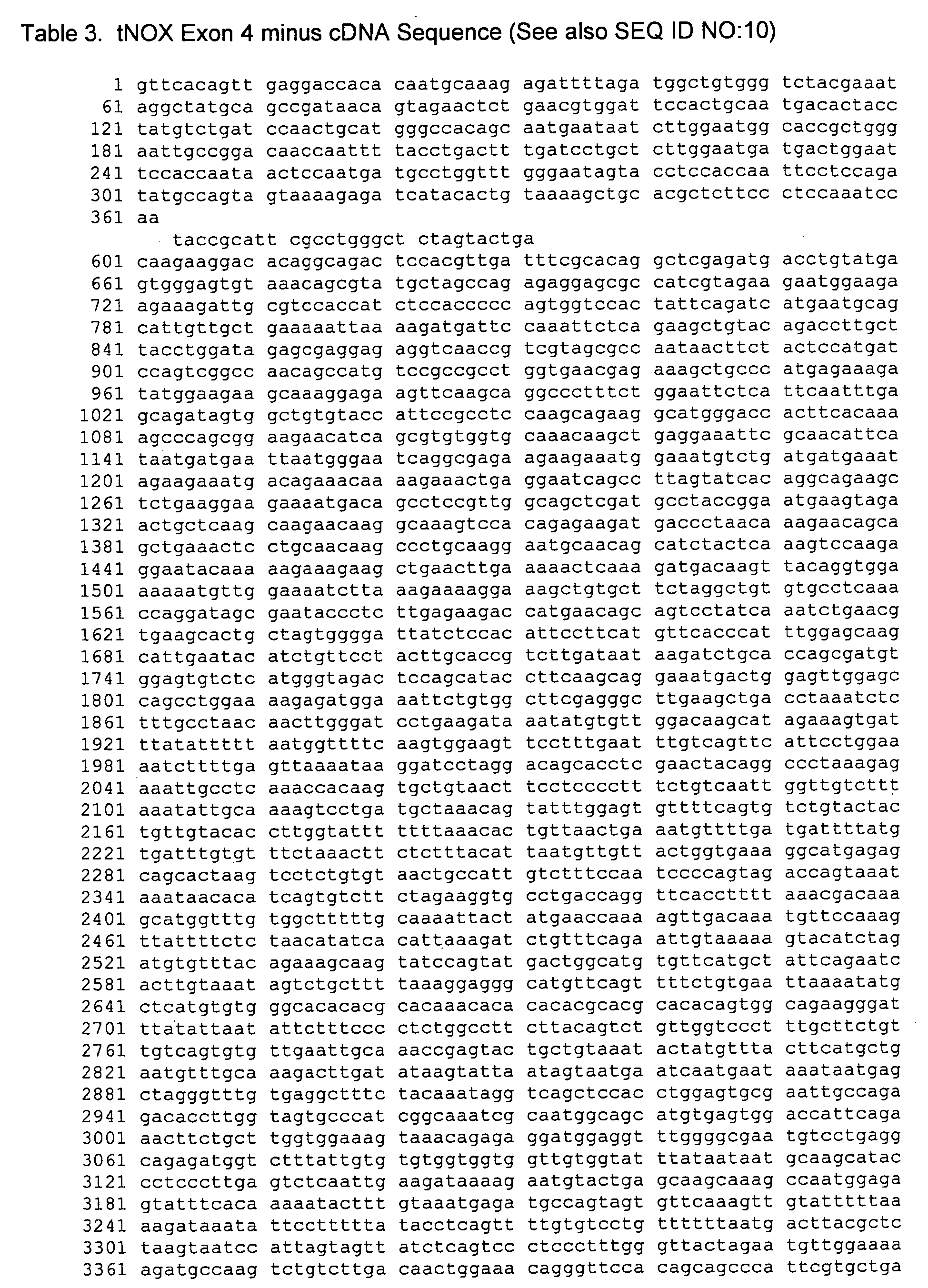

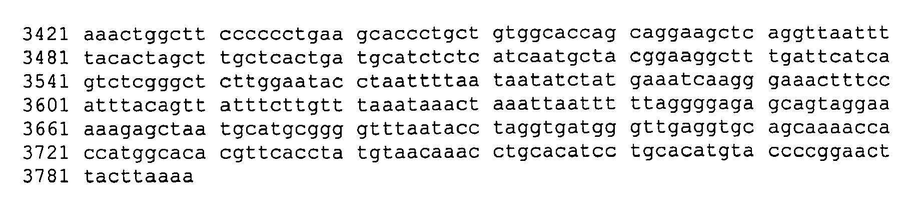

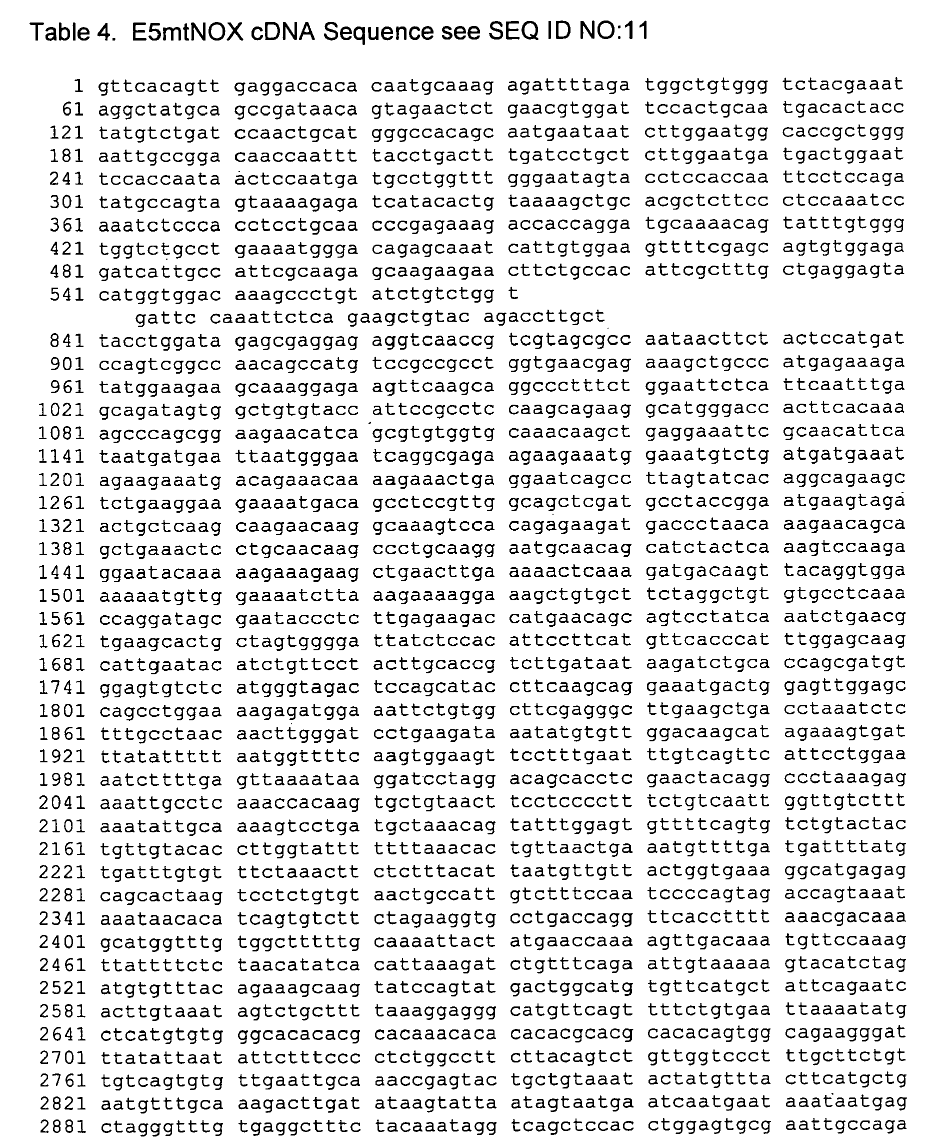

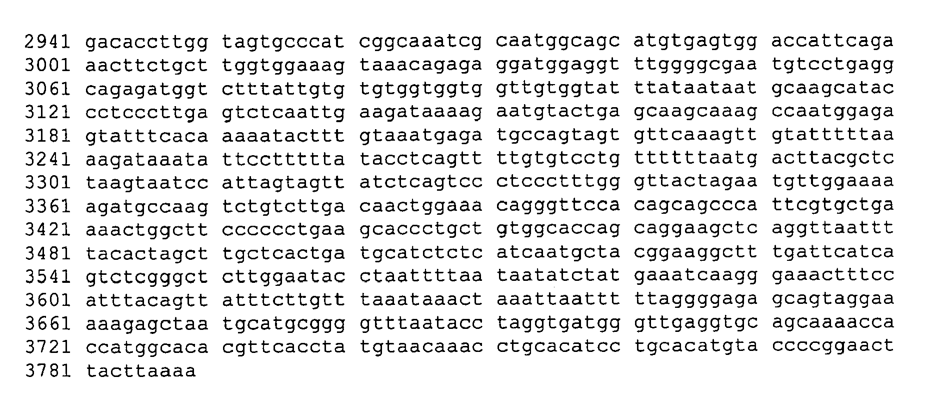

**Table 5. Antisense oligonucleotides and their target site on tNOX mRNA.**

| Oligonucleotide | Sequence (5'-3') | Position (nucleotide no.) |
|---|---|---|
| E4AS | G*T*C*CACCATGTACTCCTC*A*G*C | 530-550 |
| | (SEQ ID NO:18) | |
| E4C | A*G*C*TCTTTCCGCCCATCG*A*A*C | |
| | (SEQ ID NO:19) | |
| E5AS | G*T*C*TGCCTGTGTCCTTCT*T*G*T | 600-620 |
| | (SEQ ID NO:20) | |
| E5C | T*G*G*CGTTTCCGTTCTCGT*C*T*T | |
| | (SEQ ID NO:21) | |

| | | |
|---|---|---|
| * Phosphorothioated | | |

**Table 6. Three sets of primers used for real-time PCR of tNOX.**

| Primers | detect mRNA | Sequence(5'-3') | Position (nucleotide no.) |
|---|---|---|---|
| FLS | FL | TCG CTT TGC TGA GGA GTA CAT GGT | 523-546 |
| | | (SEQ ID NO:22) | |
| FLR | | TCT GCC TGT GTC CTT CTT GTC AGT | 596-619 |
| | | (SEQ ID NO:23) | |
| E3/5S | E4m | CCA AAT CCA ATA CCG CAT TCG C | 353-362,572-583 |
| | | (SEQ ID NO:24) | |
| E3/5R | | AGC ATA CGC TGT TTA CAC TCC CAC | 661-684 |
| | | (SEQ ID NO:25) | |
| E4/6S | E5m | CTG TCT GGT GAT TCC AAA TTC | 563-571,806-817 |
| | | (SEQ ID NO:26) | |
| E4/6R | | TTC TCA TGG GCA GCT TTC TCG TTC | 934-957 |
| | | (SEQ ID NO:27) | |

### CLAUSES

In further aspects, the present invention also provides:
1. A nucleic acid molecule of 1000 or fewer nucleotides, wherein said nucleic acid molecule comprises the nucleotide sequence as given in SEQ ID NO:4 or SEQ ID NO:6.
2. A method for detecting neoplastic cells in a biological sample, said method comprising the steps of :
   (a) providing nucleic acid extracted from a biological sample,
   (b) contacting the nucleic acid extracted from the biological sample with at least one oligonucleotide having a nucleotide sequence of SEQ ID NO:4 or SEQ ID NO:6 in a reverse transcriptase polymerase chain reaction (RT-PCR); and
   (c) detecting an amplification product of the RT-PCR,
   whereby neoplastic cells are detected in the sample when an amplification product is detected in step (c).
3. The method of clause 2, wherein the contacting step is with two oligonucleotide primers comprising nucleotide sequences of SEQ ID NO:4 and SEQ ID NO:5 and wherein the amplification product is 670 nucleotides in length.
4. A non-naturally occurring recombinant DNA molecule comprising a portion encoding an alternatively spliced NADH oxidase/protein disulfide-thiol interchange polypeptide (E4mtNOX), said portion consisting essentially of the nucleotide sequence of SEQ ID NO:10.
5. A host cell transformed or transfected to contain the recombinant DNA molecule of clause 4.
6. The host cell of clause 5 which is a bacterial cell.
7. The host cell of clause 6, wherein said bacterial cell is an *Escherichia coli* cell.
8. The host cell of clause 5, wherein said cell is a eukaryotic cell.
9. The host cell of clause 8 wherein said cell is a mammalian cell.
10. The host cell of clause 10, wherein said cell is a COS cell.
11. A method for recombinantly producing a NADH oxidase/protein disulfide-thiol interchange active polypeptide in a host cell, said method comprising the steps of:
   a) infecting or transforming a host cell with a vector comprising a promoter active in said host cell and a coding portion comprising an alternatively spliced NADH oxidase/thiol exchange protein sequence as given in SEQ ID NO:10, said promoter being operably linked to said coding portion; and
   b) culturing the recombinant host cell under conditions wherein said polypeptide is expressed.
12. A method of detecting a neoplastic condition in cells or biopsy tissue from a patient suspected of having a neoplastic condition, said method comprising the steps of:
   (a) providing nucleic acid extracted from cells or biopsy tissue taken from a patient;
   (b) contacting the nucleic acid extracted from the cells or biopsy tissue with a forward oligonucleotide primer having a nucleotide sequence of from 15 to 362 contiguous nucleotides from nucleotides 1 to 262 of SEQ ID NO:12 and with a reverse oligonucleotide primer having a nucleotide sequence complementary to from 15 to 1000 contiguous nucleotides of nucleotides 800 to 3789 of SEQ ID NO:12 in a reverse transcriptase polymerase chain reaction (RT-PCR) or contacting the nucleic acid extracted from the cells or biopsy tissue with a forward oligonucleotide primer having a nucleotide sequence of from 15 to 570 contiguous nucleotides taken from nucleotide 1 to 570 of SEQ ID NO:12 and with a reverse oligonucleotide primer complementary to from 15 to 1000 contiguous nucleotides of nucleotides 1000 to 3789 of SEQ ID NO:12 in a reverse transcriptase polymerase chain reaction (RT-PCR); and
   (c) detecting at least one amplification product of the RT-PCR,
   whereby neoplastic cells are detected in the sample when at least one amplification product is detected in step (c) which is 208 nucleotides shorter in length or 233 nucleotides shorter in length than an amplification product from SEQ ID NO:12 as template.
13. The method of clause 12, wherein said forward oligonucleotide primer and said reverse primer are from 15 to 25 nucleotides in length.
14. The method of clause 12, wherein a size separation step precedes the detecting step.
15. An antisense nucleic acid molecule which blocks tNOX protein synthesis and which restores a normal phenotype to neoplastic cells into which said antisense nucleic acid molecule has been introduced.
16. The antisense molecule of clause 15, wherein said antisense nucleic acid molecule comprise a nucleotide sequence as set forth in SEQ ID NO:18, SEQ ID NO:20, SEQ ID NO:25, or SEQ ID NO:27.

### BIBLIOGRAPHY

1. Morré, D.J. (1998) in Plasma Membrane Redox Systems and Their Role in Biological Stress and Disease (Asard, H., Bérczi, A. and Caubergs, R.J., Eds) pp. 121-156, Kluwer Academic Publishers, Dordrecht.
2. Morré, D.J. and Morré, D.M. (2003) Free Radical Res. 37: 795-808
3. Morré, D.J., Chueh, P.-J. and Morré, D.M. (1995) Proc. Natl. Acad. Sci. USA 92:1831-1835.
4. Bruno, M., Brightman, A.O., Lawrence, J., Werderitsh, D., Morré, D.M. and Morré, D.J. (1992) Biochem. J. 284: 625-628.
5. Cho, N., Chueh, P.-J., Kim, C., Caldwell, S., Morré, D.M. and Morré, D.J. (2002) Cancer Immunol. Immunother. 51: 121-129.
6. Morré, D.J., Caldwell, S., Mayorga, A., Wu, L-Y. and Morré, D.M. (1997) Arch. Biochem. Biophys. 342: 224-230.
7. Morré, D.J. and Reust, T. (1997) J. Bioenerg. Biomemb. 29, 281-289.
8. Morré, D.J. (1995) Biochim. Biophys. Acta 1240: 201-208.
9. del Castillo-Olivares, A., Chueh, P.-J., Wang, S., Sweeting, M., Yantiri, F., Sedlak, D., Morré, D.J. and Morré, D.M. (1998) Arch. Biochem. Biophys. 358:125-140
10. Morré, D.J., Sedlak, D., Tang, X., Chueh, P.J., Geng, T. and Morré, D.M. (2001) Arch. Biochem. Biophys. 392: 251-256.
11. Chueh, P.J., Kim, C., Cho, N., Morré, D.M. and Morré, D.J. (2002) Biochemistry 41: 3732-3741
12. Bird, C. (1999) Direct submission of human DNA sequence from clone 875H3 (part of APK1 antigen) to GenBank database at NCBI. (Accession no. AL049733).
13. Braman, J., Papworth, C. and Greener, A.. (1996) Methods Mol. Biol. 57: 31-44
14. Wilkinson, F.E., Kim, C., Cho, N., Chueh, P. J., Leslie, S., Moya-Camerena, S., Wu, L.-Y., Morré, D.M and Morré, D.J. (1996) Arch. Biochem. Biophys. 336: 275-282
15. Morré, D.J., Wu, L.-Y. and Morré, D.M. (1995) Biochim. Biophys. Acta 1240: 11-17.
16. Morré, D.J., Kim, C., Paulik, M., Morré, D.M. and Faulk, W.P. (1997) J. Bioenerg. Biomembr 29: 269-280.
17. Morré, D.J., Bridge, A., Wu, L.-Y. and Morré, D.M. (2000) Biochem. Pharmacol. 60: 937-946.
18. Kozak M. (1978) Cell. 15: 1109-23
19. Kozak, M. (1989) J. Cell Biol. 108: 229-241
20. Tatyana, V., Borukhov, S.I. and Hellen C. (1998) Nature 394: 854-859
21. Van der Velden, A. W. and Thomas, A. A. (1999) Int. J. Biochem. Cell Biol. 31:87-106
22. Suzuki, Y., lshihara, D., Sasaki, M. Nakagawa, H., Hata, H., Tsunoda, T., Watanabe, M., Komatsu, T., Ota, T., Isogai. T., Suyama, A., and Sugano, S. (2000) Genomics 64: 286-297
23. Kozak, M. (1987) Nucleic Acids Res. 15: 8125-8148

## Claims

1. A polynucleotide sequence comprising the sequence of SEQ ID NO:4 or SEQ ID NO:6, or sequences having at least 80% sequence homology thereto or which are capable of hybridising thereto under highly stringent conditions, the nucleotide sequence consisting of not more than 1000 nucleotides.

2. A polynucleotide according to claim 1, comprising a sequence having at least 85% sequence homology to SEQ ID NO. 4 or SEQ ID NO. 6.

3. A polynucleotide according to claim 1, comprising a sequence having at least 90% sequence homology to SEQ ID NO. 4 or SEQ ID NO. 6.

4. A polynucleotide according to claim 1, comprising a sequence having at least 95% sequence homology to SEQ ID NO. 4 or SEQ ID NO. 6.

5. A polynucleotide according to any preceding claim comprising at least 15 consecutive nucleotides from SEQ ID NO: 4 or SEQ ID NO: 6 or sequences complementary thereto that bind under highly stringent conditions.

6. A polynucleotide according to any preceding claim, wherein the highly stringent conditions include hybridizing at 68° C in 5X SSC/5X Denhardt's solution/0.1% SDS, and washing in 0.2X SSC/0.1% SDS at room temperature.

7. A polynucleotide according to any preceding claim capable of hybridizing to the splice junctions of the alternatively spliced tNOX cDNA present within SEQ ID NO: 4 or SEQ ID NO: 6.

8. A method for detecting neoplastic cells in a sample, said method comprising the steps of :
(a) providing nucleic acid extracted from a sample,
(b) contacting the nucleic acid extracted from the sample with at least one oligonucleotide having a nucleotide sequence according to any preceding claim in a reverse transcriptase polymerase chain reaction (RT-PCR); and
(c) detecting an amplification product of the RT-PCR,
whereby an amplification product is detected in step (c) is indicative of the presence of neoplastic cells in the sample.

9. The method according to claim 8, wherein the contacting step is with two oligonucleotide primers comprising nucleotide sequences of SEQ ID NO:4 and SEQ ID NO:5 and wherein the amplification product is 670 nucleotides in length.

10. A recombinant DNA molecule comprising a portion encoding an alternatively spliced NADH oxidase/protein disulfide-thiol interchange polypeptide (E4mtNOX), said portion consisting essentially of the nucleotide sequence of SEQ ID NO:10, or a sequence having at least 80% sequence homology thereto or which are capable of hybridizing thereto under highly stringent conditions.

11. A host cell transformed or transfected to contain the recombinant DNA molecule according to claim 10.

12. A host cell according to claim 11, which is a bacterial cell.

13. A host cell according to claim 12, wherein said bacterial cell is an *Escherichia coli* cell.

14. A host cell according to claim 11, wherein said cell is a eukaryotic cell.

15. A host cell according to claim 14 wherein said cell is a mammalian cell.

16. A host cell according to claim 15, wherein said cell is a COS cell.

17. A method for recombinantly producing a NADH oxidase/protein disulfide-thiol interchange active polypeptide in a host cell, said method comprising the steps of:
a) infecting or transforming a host cell ex *vivo* with a vector comprising a promoter active in said host cell and a coding portion comprising an alternatively spliced NADH oxidase/thiol exchange protein sequence as given in SEQ ID NO:10 or a sequence having at least 80% sequence homology thereto or which are capable of hybridising thereto under highly stringent conditions, said promoter being operably linked to said coding portion; and
b) culturing the recombinant host cell under conditions wherein said polypeptide is expressed.

18. A method of detecting a neoplastic condition in cells or biopsy tissue in a sample from a patient suspected of having a neoplastic condition, said method comprising the steps of:
(a) providing nucleic acid extracted from cells or biopsy tissue taken from a patient;
(b) contacting the nucleic acid extracted from the cells or biopsy tissue with a forward oligonucleotide primer having a nucleotide sequence of from 15 to 362 contiguous nucleotides from nucleotides 1 to 262 of SEQ ID NO:12 or a sequence having at least 80% sequence homology thereto or which are capable of hybridizing thereto under highly stringent conditions and with a reverse oligonucleotide primer having a nucleotide sequence complementary to from 15 to 1000 contiguous nucleotides of nucleotides 800 to 3789 of SEQ ID NO:12 or a sequence having at least 80% sequence homology thereto or which are capable of hybridising thereto under highly stringent conditions, in a reverse transcriptase polymerase chain reaction (RT-PCR) or contacting the nucleic acid extracted from the cells or biopsy tissue with a forward oligonucleotide primer having a nucleotide sequence of from 15 to 570 contiguous nucleotides taken from nucleotide 1 to 570 of SEQ ID NO:12 or a sequence having at least 80% sequence homology thereto or which are capable of hybridising thereto under highly stringent conditions, and with a reverse oligonucleotide primer complementary to from 15 to 1000 contiguous nucleotides of nucleotides 1000 to 3789 of SEQ ID NO:12 or a sequence having at least 80% sequence homology thereto or which are capable of hybridising thereto under highly stringent conditions, in a reverse transcriptase polymerase chain reaction (RT-PCR); and
(c) detecting at least one amplification product of the RT-PCR,
whereby neoplastic cells are detected in the sample when at least one amplification product is detected in step (c) which is 208 nucleotides shorter in length or 233 nucleotides shorter in length than an amplification product from SEQ ID NO:12 as template.

19. A method according to claim 18, wherein the sequences of the primers comprise at least 95% sequence homology to the respective positions of SEQ ID 12.

20. A method according to claim 19, wherein the sequences of the primers consist of the respective positions of SEQ ID 12.

21. A method according to claim 20, wherein said forward oligonucleotide primer and said reverse primer are from 15 to 25 nucleotides in length.

22. A method according to claim 21, wherein a size separation step precedes the detecting step.

23. An antisense nucleic acid molecule which blocks tNOX protein synthesis and which restores a normal phenotype to neoplastic cells into which said antisense nucleic acid molecule has been introduced.

24. The antisense molecule of claim 23, wherein said antisense nucleic acid molecule comprise a nucleotide sequence as set forth in SEQ ID NO:18, SEQ ID NO:20, SEQ ID NO:25, or SEQ ID NO:27.

25. A probe for detecting the presence of neoplastic cells in a sample, comprising a polynucleotide according to any of claim 1-6 or the DNA molecule according to claim 10.

26. A probe according to claim 25, comprising the sequence of SEQ ID NO: 4 or SEQ ID NO: 6.

27. A probe according to claim 25 or 26 comprising a detectable marker.

28. A kit for detection of neoplastic cells comprising a polynucleotide according to any of claim 1-6 or the DNA molecule according to claim 10 or probe according to any of claims 25-27.
